# EUROPEAN PATENT SPECIFICATION

(11) **EP 0 596 057 B1**
(45) Date of publication and mention of the grant of the patent: **09.09.1998**
(21) Application number: 93908010.7
(22) Date of filing: 08.04.1993
(51) Int. Cl.: C07D 453/02, A61K 31/445

(54) **QUINUCLIDINE DERIVATIVES AS SQUALENE SYNTHASE INHIBITORS**
CHINUCLIDINE DERIVATE ALS SQUALEN SYNTHASE INHIBITORE
DERIVES DE QUINUCLIDINE UTILES COMME INHIBITEURS DE SQUALENE SYNTHASE

(30) Priority: 10.04.1992 GB 9207961
(43) Date of publication of application: 11.05.1994
(73) Proprietor: ZENECA LIMITED, London W1Y 6LN (GB)
(72) Inventor: BROWN, George Robert, Wilmslow, Chesire SK9 6HH (GB); MALLION, Keith Blakeney, Knutsford, Cheshire WA16 8AE (GB); HARRISON, Peter, John, deceased (GB)
(74) Representative: Bill, Kevin
(86) International application number: GB9300743
(87) International publication number: WO9321183

(56) References cited:
- WO-A-92/15579

## Description

### Field of Invention

This invention relates to novel heterocyclic derivatives and, more particularly to novel heterocyclic derivatives which possess the pharmacologically useful property of inhibiting squalene synthase. The invention also relates to pharmaceutical compositions for use in treating diseases or medical conditions such as hypercholesterolemia and atherosclerosis, as well as other diseases and conditions in which inhibition of squalene synthase is desirable. The invention also relates to processes for the preparation of the novel heterocyclic derivatives, and to their use in medicine.

### Background to Invention

Several different classes of compounds have been reported to possess the capability of being able to lower cholesterol levels in blood plasma. For example agents which inhibit the enzyme HMG CoA reductase, which is essential for the production of cholesterol, have been reported to reduce levels of serum cholesterol. Illustrative of this class of compounds is the HMG CoA reductase inhibitor known as lovastatin which is disclosed in US Patent No 4,231,938. Other agents which are reported to lower serum cholesterol include those which act by complexing with bile acids in the intestinal system and which are hence termed "bile acid sequestrants". It is believed that many of these agents act by sequestering bile acids within the intestinal tract. This results in a lowering of the levels of bile acid circulating in the enteroheptatic system and promoting replacement of bile acids by synthesis in the liver from cholesterol, which results in an upregulation of the heptatic LDL receptor, and thus in a lowering of circulating blood cholesterol levels.

Squalene synthase (also referred to in the art as squalene synthetase) is a microsomal enzyme which catalyses the first committed step of cholesterol biosynthesis. Two molecules of farnesyl pyrophosphate (FPP) are condensed in the presence of the reduced form of nicotinamide adenine dinucleotide phosphate (NADPH) to form squalene. The inhibition of this committed step to cholesterol should leave unhindered biosynthetic pathways to ubiquinone, dolichol and isopentenyl t-RNA. Elevated cholesterol levels are known to be one of the main risk factors for ischaemic cardiovascular disease. Thus, an agent which inhibits squalene synthase should be useful in treating diseases and medical conditions in which a reduction in the level of cholesterol is desirable, for example hypercholesterolemia and atherosclerosis.

Thus far, the design of squalene synthase inhibitors has concentrated on the preparation of analogues of the substrate farnesyl pyrophosphate (FPP), and hence on compounds which contain phosphorus groups. For example, the preparation of phosphorous-containing squalene synthase inhibitors is reported in published European Patent Application No. 409,181; and the preparation of isoprenoid (phosphinylmethyl)phosphonates as inhibitors of squalene synthase is reported by Biller et al, J. Med. Chem., 1988, 31, 1869.

### Disclosure of Invention

The present invention is based on the discovery that certain heterocyclic derivatives are inhibitors of squalene synthase, and are hence useful in treating diseases and medical conditions in which inhibition of squalene synthase is desirable.

In particular the present invention is concerned with compounds of formula I (formula set out hereinafter together with the other chemical formulae referred to herein), and pharmaceutically acceptable salts thereof, wherein:
R¹ is hydrogen or hydroxy;
R² is hydrogen;
or R¹ and R² are joined together so that CR¹⁻CR² is a double bond;
X is selected from -CH₂CH₂₋, -CH=CH-, -C≡C-, -CH₂O-, -OCH₂₋, -CH₂NH-, -NHCH₂₋, -CH₂CO-, -COCH₂₋, -NHCO-, -CONH-, -CH₂S-, -SCH₂₋, wherein the sulphur atom in the latter two groups may optionally bear one or two oxygen atoms;
Ar¹ is a phenylene moiety;
Ar² is phenyl; and
wherein one or both of Ar¹ and Ar² may be optionally unsubstituted or independently substituted by one or more substituents selected from halogeno, hydroxy, amino, nitro, cyano, carboxy, carbamoyl, (1-6C)alkyl, (2-6C)alkenyl, (2-6C)alkynyl, (1-6C)alkoxy, (1-6C)alkylamino, di-[(1-6C)alkyl]amino, N-[(1-6C)alkyl]carbamoyl, N,N-di-[(1-6C)alkyl]carbamoyl, (1-6C)alkoxycarbonyl, (1-6C)alkylthio, (1-6C)alkylsulphinyl, (1-6C)alkylsulphonyl, halogeno-(1-6C)alkyl, (1-6C)alkanoyl, (1-4C)alkylenedioxy and (1-6C)alkanoylamino.

Thus, according to one aspect of the present invention there is provided a compound of formula I, or a pharmaceutically acceptable salt thereof,
wherein:
R¹ is hydrogen or hydroxy;
R² is hydrogen;
or R¹ and R² are joined together so that CR¹⁻CR² is a double bond; X is selected from -CH₂CH₂-, -CH=CH-, -C≡C-, -CH₂O-, -OCH₂₋, -CH₂NH-, -NHCH₂-, -CH₂CO-, -COCH₂-, -NHCO-, -CONH-, -CH₂S-, -SCH_{2-,} wherein the sulphur atom in the latter two groups may optionally bear one or two oxygen atoms;
Ar¹ is a 1,4-phenylene moiety;
Ar² is phenyl; and
wherein one or both of Ar¹ and Ar² may be optionally unsubstituted or independently substituted by one or more substituents selected from halogeno, hydroxy, amino, nitro, cyano, carboxy, carbamoyl, (1-6C)alkyl, (2-6C)alkenyl, (1-6C)alkoxy, (1-6C)alkylamino, di-[(1-6C)alkyl]amino, N-[(1-6C)alkyl]carbamoyl, N,N-di-[(1-6C)alkyl]carbamoyl, (1-6C)alkoxycarbonyl, (1-6C)alkylthio, (1-6C)alkylsulphinyl, (1-6C)alkylsulphonyl, halogeno-(1-6C)alkyl and (1-6C)alkanoylamino.

According to a further aspect of the present invention there is provided a compound of formula I, or a pharmaceutically acceptable salt thereof,
wherein:
R¹ is hydroxy;
R² is hydrogen;
X is selected from -CH₂CH₂-, -CH=CH-, -C≡C-, -CH₂O-, -OCH₂₋, -CH₂NH-, -NHCH₂₋, -CH₂CO-, -COCH₂₋, -NHCO-, -CONH-, -CH₂S-, -SCH₂₋, wherein the sulphur atom in the latter two groups may optionally bear one or two oxygen atoms;
Ar¹ is a phenylene moiety;
Ar² is phenyl; and
wherein one or both of Ar¹ and Ar² may be optionally unsubstituted or independently substituted by one or more substituents selected from halogeno, hydroxy, amino, nitro, cyano, carboxy, carbamoyl, (1-6C)alkyl, (2-6C)alkenyl, (2-6C)alkynyl, (1-6C)alkoxy, (1-6C)alkylamino, di-[(1-6C)alkyl]amino, N-[(1-6C)alkyl]carbamoyl, N,N-di-[(1-6C)alkyl]carbamoyl, (1-6C)alkoxycarbonyl, (1-6C)alkylthio, (1-6C)alkylsulphinyl, (1-6C) alkylsulphonyl, halogeno-(1-6C)alkyl, (1-6C)alkanoyl, (1-4C)alkylenedioxy and (1-6C)alkanoylamino.

It will be appreciated that, depending on the nature of the substituents, certain of the compounds of formula I may possess one or more chiral centres. In such circumstances, it will be appreciated that the compounds of the invention may exist in, and be isolated in, optically active or racemic form. The invention includes any optically active or racemic form of a compound of formula I which possesses the beneficial pharmacological effect of inhibiting squalene synthase. The synthesis of optically active forms may be carried out by standard techniques of organic chemistry well known in the art, for example by, resolution of a racemic form, by synthesis from optically active starting materials or by asymmetric synthesis.

It will also be appreciated that, when X is -C=C- the compounds of formula I may exist as geometric isomers. The invention also includes any such isomers which possess the beneficial pharmacological effect of inhibiting squalene synthase.
It is also to be understood that generic terms such as "alkyl" include both the straight chain and branched chain groups such as butyl and tert-butyl. However, when a specific term such as "butyl" is used, it is specific for the straight chain or "normal" butyl group, branched chain isomers such as "t-butyl" being referred to specifically when intended.

It will be appreciated that when R¹ and R² are joined so that CR¹⁻CR² is a double bond, the heterocyclic ring in formula I will comprise the 2,3-dehydroquinuclidine moiety shown in formula Ia.

A particular value for an alkyl substituent which may be present on Ar¹ or Ar² is, for example, (1-4C)alkyl, such as methyl, ethyl, propyl, isopropyl, butyl, isobutyl or sec-butyl.

A particular value for an alkenyl substituent which may be present on Ar¹ or Ar² is, for example, (2-4C)alkenyl, such as allyl, but-2-enyl or 2-methyl-2-propenyl.

A particular value for an alkynyl substituent which may be present on Ar¹ or Ar² is, for example, (2-4C)alkynyl, such as prop-2-ynyl or but-2-ynyl.

A particular value for an alkoxy substituent which may be present on Ar¹ or Ar² is, for example, (1-4C)alkoxy, such as methoxy, ethoxy, propoxy, isopropoxy or butoxy.

A particular value for an alkylamino substituent which may be present on Ar¹ or Ar² is, for example, (1-4C)alkylamino, such as methylamino, ethylamino, propylamino or butylamino.

A particular value for a di-alkylamino substituent which may be present on Ar¹ or Ar² is, for example, dimethylamino, diethylamino, methylpropylamino or dipropylamino.

A particular value for an alkylcarbamoyl substituent which may be present on Ar¹ or Ar² is, for example, N-methylcarbamoyl, N-ethylcarbamoyl or N-propylcarbawoyl.

A particular value for a di-alkylcarbamoyl substituent which may be present on Ar¹ or Ar² is, for example, N,N-dimethylcarbamoyl or N,N-diethylcarbamoyl.

A particular value for an alkoxycarbonyl substituent which may be present on Ar¹ or Ar² is, for example, methoxycarbonyl, ethoxycarbonyl or propoxycarbonyl.

A particular value for an alkylthio substituent which may be present on Ar¹ or Ar² is, for example, methylthio, ethylthio, propylthio, isopropylthio or butylthio.

A particular value for an alkylsulphinyl substituent which may be present on Ar¹ or Ar² is, for example, methylsulphinyl, ethylsulphinyl, propylsulphinyl, isopropylsulphinyl or butylsulphinyl.

A particular value for an alkylsulphonyl substituent which may be present on Ar¹ or Ar² is, for example, methylsulphonyl, ethylsulphonyl, propylsulphonyl, isopropylsulphonyl or butylsulphonyl.

A particular value for a halogeno substituent which may be present on Ar¹ or Ar² is, for example, fluoro, chloro or bromo.

A particular value for a halogenoalkyl substituent which may be present on Ar¹ or Ar² is, for example, one which contains one, two or three halo groups selected from fluoro, chloro and bromo and the alkyl group is selected from methyl, ethyl, propyl, iso-propyl, butyl, isobutyl or sec-butyl (in particular fluoromethyl, difluoromethyl or trifluoromethyl).

A particular value for an alkanoyl substituent which may be present on Ar¹ or Ar² is, for example, formyl, acetyl, propionyl and butyryl.

A particular value for an alkanoylamino substituent which may be present on Ar¹ or Ar² is, for example, formamido, acetamido or propionamido.

A particular value for alkylenedioxy is, for example, methylenedioxy and isopropylidenedioxy.

A particular value for Ar¹ is, for example, an optionally substituted 1,3-phenylene or 1,4-phenylene moiety.

A particular value of X is one selected from -CH₂CH₂-, -CH=CH-, -C=C-, -CH₂O-, -OCH₂-, -CONH- and -NHCO-.

Further particular values of X include, for example, -CH₂CH₂-, -CH=CH-, -C≡C-, -CH₂O- and -OCH₂-.

A more particular value for Ar¹ is an unsubstituted 1,4-phenylene moiety, and for Ar² is a phenyl moiety which is unsubstituted or bears one, two ot three substituents selected from those defined above.

In general, it is preferred that Ar¹ comprises a 1,4-phenylene moiety (optipnally substituted as defined above).

In general, it is preferred that R¹ is hydroxy and R² is hydrogen.

In general it is preferred, for example, that one or both of Ar¹ and Ar² are unsubstituted or substituted by one or more substituents independently selected from halogeno, hydroxy, nitro, cyano, (1-6C)alkyl, (2-6C)alkenyl, (1-6C)alkoxy, (1-6C)alkylthio, (1-6C)alkylsulphinyl, (1-6C)alkylsulphonyl, halogeno-(1-6C)alkyl, (1-6C)alkanoylamino and (1-4C)alkylenedioxy.

In general, it is preferred that each of Ar¹ and Ar² is unsubstituted or bears up to three substituents.

Specific values for X include -CH₂CH₂-,-CH=CH-, -C≡C-, -OCH₂-, -CH₂O- and -NHCO-.

A specific value for Ar¹ is an unsubstituted 1,4-phenylene moiety.

A specific value for Ar² is a phenyl moiety which is unsubstituted or which bears an alkoxy (such as a 4-methoxy), a (1-4C)alkylenedioxy (such as a 3,4-methylenedioxy) or a hydroxy (such as a 4-hydroxy) group.

In a specific example, the phenyl rings (Ar¹ and Ar²) are both unsubstituted.

In one embodiment of the present invention R¹ is hydrogen; R² is hydrogen; in a further embodiment R¹ is is hydroxy and R² is hydrogen; and in a further embodiment R¹ and R² are joined together so that CR¹-CR² is a double bond; and in each case X, Ar¹ and Ar² are as hereinbefore defined.

In a further embodiment of the present invention R¹ is hydroxy; R² is hydrogen; X is selected from -CH₂CH₂-, -CH=CH-, -C≡C-, -CH₂O-, -OCH₂-, -CH₂NH-, -NHCH₂-, -CH₂CO-, -COCH₂-, -NHCO-, -CONH-, -CH₂S-, -SCH₂-, wherein the sulphur atom in the latter two groups may optionally bear one or two oxygen atoms; Ar¹ is a 1,4-phenylene moiety; Ar² is phenyl; and wherein one or both of Ar¹ and Ar² may be optionally unsubstituted or independently substituted by one or more substituents selected from halogeno, hydroxy, amino, nitro, cyano, carboxy, carbamoyl, (1-6C)alkyl, (2-6C)alkenyl, (2-6C)alkynyl, (1-6C)alkoxy, (1-6C)alkylamino, di-[(1-6C)alkyl]amino, N-[(1-6C)alkyl]carbamoyl, N,N-di-[(1-6C)alkyl]carbamoyl, (1-6C)alkoxycarbonyl, (1-6C)alkylthio, (1-6C)alkylsulphinyl, (1-6C)alkylsulphonyl, halogeno-(1-6C)alkyl, (1-6C)alkanoyl, (1-4C)alkylenedioxy and (1-6C)alkanoylamino.

Particular, preferred and specific values are those mentioned above.

In a further embodiment R¹ and R² are joined together so that CR¹-CR² is a double bond; X is selected from -CH₂CH₂-, -CH=CH-, -C≡C-, -CH₂O-, -OCH₂-, -CH₂NH-, -NHCH₂-, -CH₂CO-, -COCH₂-, -NHCO-, -CONH-, -CH₂S-, -SCH₂-, wherein the sulphur atom in the latter two groups may optionally bear one or two oxygen atoms; Ar¹ is a 1,4-phenylene moiety; Ar² is phenyl; and
wherein one or both of Ar¹ and Ar² may be optionally unsubstituted or independently substituted by one or more substituents selected from halogeno, hydroxy, amino, nitro, cyano, carboxy, carbamoyl, (1-6C)alkyl, (2-6C)alkenyl, (2-6C)alkynyl, (1-6C)alkoxy, (1-6C)alkylamino, di-[(1-6C)alkyl]amino, N-[(1-6C)alkyl]carbamoyl, N,N-di-[(1-6C)alkyl]carbamoyl, (1-6C)alkoxycarbonyl, (1-6C)alkylthio, (1-6C)alkylsulphinyl, (1-6C)alkylsulphonyl, halogeno-(1-6C)alkyl, (1-6C)alkanoyl, (1-4C)alkylenedioxy and (1-6C)alkanoylamino.

Particular, preferred and specific values are those mentioned above.

In a further embodiment R¹ is hydrogen; R² is hydrogen; X is selected from -CH₂CH₂-, -CH=CH-, -C≡C-, -CH₂O-, -OCH₂-, -CH₂NH-, -NHCH₂-, -CH₂CO-, -COCH₂-, -NHCO-, -CONH-, -CH₂S-, -SCH₂-, wherein the sulphur atom in the latter two groups may optionally bear one or two oxygen atoms; Ar¹ is a 1,4-phenylene moiety; Ar² is phenyl; and wherein one or both of Ar¹ and Ar² may be optionally unsubstituted or independently substituted by one or more substituents selected from halogeno, hydroxy, amino, nitro, cyano, carboxy, carbamoyl, (1-6C)alkyl, (2-6C)alkenyl, (2-6C)alkynyl, (1-6C)alkoxy, (1-6C)alkylamino, di-[(1-6C)alkyl]amino, N-[(1-6C)alkyl]carbamoyl, N,N-di-[(1-6C)alkyl]carbamoyl, (1-6C)alkoxycarbonyl, (1-6C)alkylthio, (1-6C)alkylsulphinyl, (1-6C)alkylsulphonyl, halogeno-(1-6C)alkyl, (1-6C)alkanoyl, (1-4C)alkylenedioxy and (1-6C)alkanoylamino.

Particular, preferred and specific values are those mentioned above.

In a preferred embodiment of the present invention R¹ is hydroxy; R² is hydrogen; X is selected from -CH₂CH₂-, -CH=CH-, -C≡C-, -CH₂O-, -OCH₂- and -NHCO-; Ar¹ is a 1,4-phenylene moiety; Ar² is phenyl; and
wherein one or both of Ar¹ and Ar² may be optionally unsubstituted or independently substituted by one or more substituents selected from halogeno, hydroxy, amino, nitro, cyano, carboxy, carbamoyl, (1-6C)alkyl, (2-6C)alkenyl, (2-6C)alkynyl, (1-6C)alkoxy, (1-6C)alkylamino, di-[(1-6C)alkyl]amino, N-[(1-6C)alkyl]carbamoyl, N,N-di-[(1-6C)alkyl]carbamoyl, (1-6C)alkoxycarbonyl, (1-6C)alkylthio, (1-6C)alkylsulphinyl, (1-6C)alkylsulphonyl, halogeno-(1-6C)alkyl, (1-6C)alkmoyl, (1-4C)alkflenedioxy and (1-6C)alkanoylamino.

Particular, preferred and specific values are those mentioned above.

In a particular embodiment of the present invention there is provided a compound of formula I, or a pharmaceutically acceptable salt thereof, wherein:
R¹ is hydrogen or hydroxy; R² is hydrogen; or R¹ and R² are joined together so that CR¹-CR² is a double bond; X is selected from -CH₂CH₂-, -CH-CH-, -C≡C-, -CH₂O-, -OCH₂-, -CH₂NH-, -NHCH₂-, -CH₂CO-, -COCH₂-, -CH₂S-, -SCH₂-, wherein the sulphur atom in the latter two groups may optionally bear one or two oxygen atoms; Ar¹ is a 1,4-phenylene moiety; Ar² is phenyl; and
wherein one or both of Ar¹ and Ar² may be optionally unsubstituted or independently substituted by one or more substituents selected from halogeno, hydroxy, amino, nitro, cyano, carboxy, carbamoyl, (1-6C)alkyl, (2-6C)alkenyl, (1-6C)alkoxy, (1-6C)alkylamino, di-[(1-6C)alkyl]amino, N-[(1-6C)alkyl]carbamoyl, N,N-di-[(1-6C)alkyl]carbamoyl, (1-6C)alkoxycarbonyl, (1-6C)alkylthio, (1-6C)alkylsulphinyl, (1-6C)alkylsulphonyl, halogeno-(1-6C)alkyl, and (1-6C)alkanoylamino.

Particular, preferred and specific values are those mentioned above.

In a further embodiment of the present invention R¹ and R² are both hydrogen; X is selected from -CH₂CH₂-, -CH=CH-, -C≡C-, -CH₂O-, -OCH₂-, -CH₂NH-, -NHCH₂-, -CH₂CO-, -COCH₂-, -CH₂S-, -SCH₂-, wherein the sulphur atom in the latter two groups may optionally bear one or two oxygen atoms; Ar¹ is a 1,4-phenylene moiety; Ar² is phenyl; and wherein one or both of Ar¹ and Ar2 may be optionally unsubstituted or independently substituted by one or more substituents selected from halogeno, hydroxy, amino, nitro, cyano, carboxy, carbamoyl, (1-6C)alkyl, (2-6C)alkenyl, (1-6C)alkoxy, (1-6C)alkylamino, di-[(1-6C)alkyl]amino, N-[(1-6C)alkyl]carbamoyl, N,N-di-[(1-6C)alkyl]carbamoyl, (1-6C)alkoxycarbonyl, (1-6C)alkylthio, (1-6C)alkylsulphinyl, (1-6C)alkylsulphonyl and halogeno-(1-6C)alkyl and (1-6C)alkanoylamino.

Particular, preferred and specific values are those mentioned above.

In a further embodiment R¹ is hydroxy and R² is hydrogen; X is selected from -CH₂CH₂-, -CH=CH-, -C≡C-, -CH₂O-, -OCH₂-, -CH₂NH-, -NHCH₂-, -CH₂CO-, -COCH₂-, -CH₂S-, -SCH₂-, wherein the sulphur atom in the latter two groups may optionally bear one or two oxygen atoms; Ar¹ is a 1,4-phenylene moiety; Ar² is phenyl; and wherein one or both of Ar¹ and Ar² may be optionally unsubstituted or independently substituted by one or more substituents selected from halogeno, hydroxy, amino, nitro, cyano, carboxy, carbamoyl, (1-6C)alkyl, (2-6C)alkenyl, (1-6C)alkoxy, (1-6C)alkylamino, di-[(1-6C)alkyl]amino, N-[(1-6C)alkyl]carbamoyl, N,N-di-[(1-6C)alkyl]carbamoyl, (1-6C)alkoxycarbonyl, (1-6C)alkylthio, (1-6C)alkylsulphinyl, (1-6C)alkylsulphonyl, halogeno-(1-6C)alkyl and (1-6C)alkanoylamino.

Particular, preferred and specific values are those mentioned above.

In a further embodiment R¹ and R² are joined together so that CR¹-CR² is a double bond;
X is selected from -CH₂CH₂-, -CH=CH-, -C≡C-,-CH₂O-, -OCH₂-, -CH₂NH-, -NHCH₂-, -CH₂CO-, -COCH₂-, -CH₂S-, -SCH₂-, wherein the sulphur atom in the latter two groups may optionally bear one or two oxygen atoms; Ar¹ is a 1,4-phenylene moiety; Ar² is phenyl; and
wherein one or both of Ar¹ and Ar² may be optionally unsubstituted or independently substituted by one or more substituents selected from halogeno, hydroxy, amino, nitro, cyano, carboxy, carbamoyl, (1-6C)alkyl, (2-6C)alkenyl, (1-6C)alkoxy, (1-6C)alkylamino, di-[(1-6C)alkyl]amino, N-[(1-6C)alkyl]carbamoyl, N,N-di-[(1-6C)alkyl]carbamoyl, (1-6C)alkoxycarbonyl, (1-6C)alkylthio, (1-6C)alkylsulphinyl, (1-6C)alkylsulphonyl, halogeno-(1-6C)alkyl and (1-6C)alkanoylamino.

Particular, preferred and specific values are those mentioned above.

In a particular embodiment of the present invention there is provided a compound of formula I, or a pharmaceutically acceptable salt thereof, wherein:
R¹ is hydrogen or hydroxy; R² is hydrogen; or R¹ and R² are joined together so that CR¹-CR² is a double bond; X is selected from -CH₂CH₂-, -CH-CH-, -C≡C-, -CH₂O-, -OCH₂-; Ar¹ is a 1,4-phenylene moiety; Ar² is phenyl; and
wherein one or both of Ar¹ and Ar² may be optionally unsubstituted or independently substituted by one or more substituents selected from halogeno, hydroxy, amino, nitro, cyano, carboxy, carbamoyl, (1-6C)alkyl, (2-6C)alkenyl, (1-6C)alkoxy, (1-6C)alkylammo, di-[(1-6C)alkyl]amino, N-[(1-6C)alkyl]carbamoyl, N,N-di-[(1-6C)alkyl]carbamoyl, (1-6C)alkoxycarbonyl, (1-6C)alkylthio, (1-6C)alkylsulphinyl, (1-6C)alkylsulphonyl, halogeno-[1-6C)alkyl and (1-6C)alkanoyl amino.

Particular and preferred values for the various groups are the appropriate values mentioned above.

In a further embodiment of the present invention there is provided a compound of formula I, or a pharmaceutically acceptable salt thereof, wherein:
R¹ is hydrogen or hydroxy; R² is hydrogen; or R¹ and R² are joined together so that CR¹-CR² is a double bond; X is selected from -CH₂CH₂-, -CH=CH-, -C≡C-, -OCH₂-, and -CH₂O-; Ar¹ is a 1,4-phenylene moiety; Ar² is phenyl; and wherein one or both of Ar¹ and Ar² may -optionally be unsubstituted or substituted by one or more substituents selected from halogeno, hydroxy, nitro, (1-6C)alkyl, (2-6C)alkenyl, (1-6C)alkoxy, (1-6C)alkylthio, (1-6C)alkylsulphinyl, (1-6C)alkylsulphonyl and halogeno-(1-6C)alkyl.

Particular and preferred values for the various groups are the appropriate values mentioned above.

In an embodiment of particular interest R¹ is hydrogen or hydroxy; R² is hydrogen; or R¹ and R² are joined together so that CR¹-CR² is a double bond; X is selected from -CH₂CH₂-, -CH=CH-, -C≡C-, -OCH₂-, and -CH₂O-; Ar¹ is a 1,4-phenylene moiety; Ar² is phenyl; and Ar¹ and Ar² are both unsubstituted.

Compounds of the invention which are of particular interest include the compounds described in the accompanying Examples (and their pharmaceutically-acceptable salts), and are hence provided as a further feature of the present invention.

A suitable pharmaceutically-acceptable salt of the present invention comprises an acid-addition salt derived from an inorganic or organic acid which provides a pharmaceutically-acceptable anion. Thus, examples of salts of the present invention include acid-addition salts with hydrochloric, hydrobromic, nitric, sulphuric, phosphoric, trifluoroacetic, citric, tartaric, succinic, maleic, fumaric or acetic acid. In addition, suitable pharmaceutically-acceptable salts include [where the compound of formula I is sufficiently acidic, for example where the compound of fomula I bears an acidic substituent such as carboxy] those formed with a base which affords a pharmaceutically acceptable cation. Suitable bases include an alkali metal salt (such as a sodium or potassium salt), an alkaline earth metal salt (such as a calcium or magnesium salt), an ammonium salt or a salt with an organic base which affords a physiologically-acceptable cation such as a salt with methylamine, dimethylamine, triethylamine, piperidine or morpholine.

The compounds of the present invention may be obtained by standard procedures of organic chemistry already known to be applicable to the preparation of structurally analogous compounds. Such procedures for the preparation of the compounds of formula I, or pharmaceutically acceptable salts thereof, are provided as a further feature of the present invention and are illustrated by the following preferred processes in which the various generic radicals, for example, R¹, R², X, Ar¹ and Ar² may take any of the meanings hereinbefore defined.

Thus, according to the present invention there is also provided a process for preparing a compound of formula I, or a pharmaceutically-acceptable salt thereof, which process comprises:
(a) For those compounds of formula I in which R¹ and R² are both hydrogen, reducing a compound of formula I in which R¹ and R² are joined together so that CR¹-CR² is a double bond.
   The reduction may be carried out, for example, by catalytic hydrogenation, or by reaction with a suitable reducing agent. Suitable reaction conditions include, for example, catalytic hydrogenation using a catalyst which comprises a noble metal. Particular catalysts include palladium, platinum and nickel (especially when in the finely divided state known as Raney nickel), and catalysts in which the noble metal is supported on an inert carrier such as carbon. A specific example of a supported catalyst is Pd/C. The reduction is conveniently carried out in a solvent of, for example, an alcohol such as ethanol, and at (or near) ambient temperature and optionally under pressure.
   Further suitable reaction conditions include, for example, reduction with a borane such as diborane. The reaction is generally carried out in an inert solvent of, for example, tetrahydrofuran or methyl t-butyl ether at, for example, 0-60°C. It may be preferable to cool the reaction below ambient temperature (eg. to about 0°C) during the reduction. The borane generated may be hydrolysed by treatment with an organic acid such as acetic acid, which hydrolysis may be carried out at 0-60°C, and may be accelerated by heating (eg. refluxing).
(b) For compounds of formula I in which R¹ and R² are joined together so that CR¹-CR² is a double bond, dehydrating a compound of formula I in which R¹ is hydroxy and R² is hydrogen.
   The dehydration may be carried out using an acid such as sulphuric acid (eg. concentrated sulphuric acid), or p-toluene sulphonic acid. The reaction is conveniently carried out with heating, and conveniently an inert solvent is employed. For example, the reaction may be carried outusing sulphuric acid at temperatures of about 70-130°C; or using p-toluene sulphonic acid in a hydrocarbon solvent of, for example, toluene or xylene at ambient temperature to reflux, and preferably at reflux. The dehydration may also be carried out using trifluoroacetic acid in an inert solvent such as dichloromethane (at ambient temperature to reflux temperature).
(c) For compounds of formula I in which R¹ and R² are joined together so that CR¹-CR² is a double bond, treating a compound of formula II in which Z is a leaving group with a base.
   Suitable values for Z include, for example, halogen such as chloro, bromo, iodo, or a methylsulphonyloxy or toluenesulphonyloxy group. Suitable bases include hydroxide (such as potassium or sodium hydroxide), and alkoxide (such as potassium t-butoxide or sodium ethoxide).
   The reaction is conveniently carried out in the presence of a solvent, preferably a polar organic solvent. Suitable solvents include, for example, an alcohol (such as ethanol), or an aprotic solvent such as dimethylformamide or N-methylpyrrolidone. The reaction may be carried out at ambient temperature or at an elevated temperature, such as at a temperature between ambient and the reflux temperature of the reaction mixture. This method is generally preferred over that described in (b) when X is -OCH₂- or -SCH₂-.
   The compounds of formula II may be prepared from a compound of formula I in which R¹ is hydroxy. For example, where Z is halogen the compound of formula I in which R¹ is hydroxy and R² is hydrogen may be reacted with the appropriate phosphorous halide (eg. PCl₅, PBr₃ or PI₃), or where Z is chloro, by reaction with thionyl chloride. The compound of formula I in which R¹ is hydroxy may be reacted with mesyl chloride to the compound in which Z is methylsulphonyloxy; and with tosyl chloride to give Z is toluene sulphonyloxy.
(d) For those compounds in which R¹ is hydroxy and R² is hydrogen, reacting an organometallic derivative of formula III in which M is a metal atom or a derivative thereof, with quinuclidin-3-one.
   Suitable values for M include, for example, magnesium and lithium. In the case where M is magnesium it is conveniently present in the form of a derivative of formula -MgX where X is a halogen atom such as iodo or bromo, so that the organometallic compound of formula III is in the form known as a Grignard Reagent. The reaction is generally carried out in an inert solvent such as dry diethyl ether or tetrahydrofuran and, when M is lithium, with cooling. For example, when M is lithium the reaction may be carried out at a temperature below 0°C, such as at a temperature between 0°C and -78°C. When the compound of formula III is in the form of a Grignard reagent the reaction is generally carried out with heating at, for example, reflux.
   In cases where X is -COCH₂₋ or -CH₂CO- the carbonyl group may be protected as, for example, a ketal during the reaction of the compound of formula III with quinuclidin-3-one. The protecting group may then be removed using methods well known in the art.
   The compounds of formula III may be prepared from a compound of formula IIIa in which "hal" is a halogen atom, such as iodo or bromo. The compound of formula IIIa may be reacted directly with the metal M. Thus in the case of magnesium, the Grignard Reagent of formula III may be prepared by reaction of a compound of formula IIIa in which "hal" is bromo or iodo with magnesium turnings in an inert solvent such as diethyl ether, as is well known in the art. Where M is lithium, the compound of formula III may be prepared by reaction of the compound of formula IIIa with lithium in an inert solvent such as diethyl ether, or by reaction with an alkyl lithium derivative such as sec-butyl lithium in an inert solvent such as diethyl ether or tetrahydrofuran, as is well known in the art.
   The compounds of formula IIIa are generally available or may be readily prepared by procedures well known in the art. -For example those compounds of formula IIIa in which X is -CH₂O- may be prepared by reaction of a phenol with a halobenzylhalide in the presence of a base (see scheme la). It will be appreciated that the phenyl ring in the phenol and the halo benzyl halide will be unsubstituted or substituted as appropriate. Thus in a specific example of this reaction, phenol may be reacted with 4-bromobenzylbromide in the presence of potassium carbonate in a solvent such as methylethylketone, preferably with heating to about 80°C. Compounds of formula IIIa in which X is -CH₂S- may be prepared in an analogous fashion. Thus compounds of formula IIIa in which X is -CH₂S- may be prepared by reaction of thiophenol with a bromobenzylbromide in the presence of a base such as sodium hydride (see scheme la).
   Compounds of formula IIIa in which X is -OCH₂- and -SCH₂- may be prepared in an analogous way (see scheme 1b). Thus when X is -CH₂O- an appropriately substituted halophenol may be reacted with an appropriately substituted benzylhalide, for example, 4-bromophenol may be reacted with benzylbromide in the presence of potassium carbonate to give the compound of formula IIIa in which Ar and Ar² are unsubstituted. Compounds of formula IIIa which X is -SCH₂- may be prepared by reaction of a 4-bromothiophenol with benzyl bromide in the presence of a base such as sodium hydride and in a solvent such as dimethylformamide(see scheme 1b).
   Compounds of formula IIIa in which X is -CH=CH- may be prepared by reaction of an appropriately substituted benzaldehyde with benzyltriphenyl phosphonium chloride (see scheme 1c) under conditions known to those skilled in the art for carrying out Wittig reactions, such as in the presence of potassium t-butoxide in a solvent such as tetrahydrofuran and with cooling.
   Compounds of formula IIIa in which X is -CH₂CO- may be prepared by reaction of an appropriately substituted benzene derivative with an appropriately substituted acyl halide as shown in scheme 1d. The carbonyl group may then be protected by reaction with an ortho formate such as (MeO)₃CH.
   Compounds of formula IIIa in which X is -COCH₂- may be prepared by an appropriately substituted di-bromobenzene derivative with a benzylnitrile derivative as shown in scheme le. The carbonyl group may then be protected by reaction with an alcohol.
   Compounds of formula IIIa in which X is -C≡C- may be prepared by reaction of a dibromobenzene derivative with an acetylene compound (see scheme 1h) using similar conditions to those described in (f) below.
   Compounds of formula IIIa in which X is -CH₂N- and -NCH₂- may be prepared by reaction of the appropriate aniline derivative and benzaldehyde derivative, followed by reduction as indicated in scheme 1f and 1g respectively.
   Compounds of formula IIIa in which X is -NHCO- and -CONH- may be prepared by reaction of the appropriate amine with a carboxylic acid derivative such as an acyl chloride as shown in scheme 1i or 1j.
e) For those compounds of formula I in which X is -CH=CH-, reacting a compound of formula IV in which Y is -CHO with a compound of formula V in which W is a halogen atom (such as chloro), in the presence of a base.
   Suitable bases include alkoxides such-as potassium t-butoxide and the reaction is conveniently carried out in an inert solvent -such as tetrahydrofuran with cooling below ambient temperature.
   The compounds of formula V may be prepared by reaction of the appropriate benzylhalide with triphenylphosphine.
f) For those compounds of formula I in which X is -C≡C-, reacting a compound of formula IV in which Y is a leaving group, with a compound of formula VI in the presence of a catalyst.
   Suitable leaving groups include, for example, halogen such as bromo, iodo, or triflate.
   Suitable catalysts include CuI and palladium derivatives such as Pd(Ph₃P)₂Cl₂. Thus, for example the compound of formula VI may be treated with Pd(Ph₃P)₂Cl₂ in a solvent such as dimethylformamide and in the presence of a base such as triethylamine.
g) For those compounds of formula I in which X is -OCH₂-, -NHCH₂- or-SCH₂-, reacting a compound of formula IV in which Y is -OH, -NH₂ or -SH with a compound of formula VII in which Z is a leaving group, in the presence of a base.
   Suitable values for Z include halogen such as chloro, bromo or iodo; whilst suitable bases include alkali metal hydroxides such as sodium hydroxide, alkali or alkaline earth metal carbonate such as potassium carbonate and metal hydrides such as sodium hydride. The reaction is generally carried out in a solvent such as methylethylketone or dimethylformamide.
h) For those compounds of formula I in which X is -CH₂O-, -CH₂NH- or -CH₂S- by reaction a compound of formula IV in which Y is -CH₂OH, -CH₂NH₂ or -CH₂SH with a compound of formula VIII in which Z is a leaving group, in the presence of a base.
   Suitable values for Z include, for example, triflate, chloro, bromo and iodo. Suitable bases include, for example sodium hydride or alkoxides such as sodium butoxide. The reaction is generally carried out in a solvent such as dimethylformamide.
   The compounds of formula IV may be prepared by reaction of the corresponding compound of formula Y-Ar¹-M in which Ar¹ represents a phenyl moiety (optionally substituted) and M represents a metal atom or derivative such as Lithium or -MgBr, with quinuclidin-3-one. The reaction is generally carried out in an inert solvent such as diethyl ether or tetrahydrofuran, and the compoud of formula Y-Ar¹-M may be prepared by reaction of the corresponding bromo compound of formula Y-Ar¹Br with the metal. When the group Y is an -OH or -CH₂OH group it is generally protected during the reaction as, for example a silyl derivative; whilst when Y is -CHO it is generally protected as an acetal.
i) For those compounds of formula I in which X is -CH₂CH₂-, reducing a compound of formula I in which X is -C≡C- or -CH=CH-.
   The reduction may be carried out by, for example, catalytic hydrogenation or by reaction with a suitable reducing agent. Suitable reaction conditions and reagents include those described under (a) above.
j) For those compounds of formula I in which X is -CH=CH-, reacting a compound of formula IV in which Y is a leaving group with a compound of formula IX, in the presence of a catalyst.
   Suitable values for Y and the catalyst and suitable reaction conditions are these mentioned in (f) above.
   It will be appreciated that in some of the reactions mentioned herein it may be necessary/desirable to protect any sensitive groups in the compounds. The instances where protection is necessary or desirable and suitable methods for protection are known to those skilled in the art. Thus, if reactants include groups such as amino, carboxy or hydroxy it may be desirable to protect the group in some of the reactions mentioned herein. Suitable protecting groups for hydroxy include, for example, silyl groups such as trimethylsilyl or t-butyldimethylsilyl, tetrahydropyranyl and esterifing groups such as a methyl or ethyl ester; and for amino groups include benzyloxycarbonyl and t-butoxycarbonyl. Carboxy groups may be protected in a reduced form such as in the form of the corresponding protected alcohol, which may be subsequently oxidized to give the carboxy group. The protecting groups may be removed at any convenient stage in the synthesis using conventional techniques well known in the chemical art.

It will also be appreciated that the preferred process for preparing a particular compound of formula I will depend upon the nature of the various radicals. Similarly, the preferred choice of reagent will depend upon the nature of the various radicals present. For example, when it is required to reduce a particular compound the reducing agent will generally be selected to be one which does not interfere with other groupings present.

It will also be appreciated that certain of the various optional substituents in the compounds of the present invention may be introduced by standard aromatic substitution reactions or generated by conventional functional group modifications either prior to or immediately following the processes mentioned above, and as such are included in the process aspect of the invention. Such reactions and modifications include, for example, introduction of a substituent by means of an aromatic substitution reaction, reduction of substituents, alkylation of substituents and oxidation of substituents. The reagents and reaction conditions for such procedures are well known in the chemical art. Particular examples of aromatic substitution reactions include the introduction of a nitro group using concentrated nitric acid, the introduction of an acyl group using, for example, an acylhalide and Lewis acid (such as aluminium trichloride) under Friedel Crafts conditions; the introduction of an alkyl group using an alkyl halide and Levis acid (such as aluminium trichloride) under Friedel Crafts conditions; and the introduction of a halogeno group. Particular examples of modifications include the reduction of a nitro group to an amino group by for example, catalytic hydrogenation with a nickel catalyst or treatment with iron in the presence of hydrochloric acid with heating; oxidation of alkylthio to alkylsulphinyl or alkylsulphonyl.

When a pharmaceutically-acceptable salt of a compound of the formula I is required, it may be obtained, for example, by reaction of said compound with the appropriate acid (which affords a physiologically acceptable anion), or with the appropriate base (which affords a physiologically acceptable cation), or by any other conventional salt formation procedure.

As mentioned previously, the compounds of the formula I (and their pharmaceutically-acceptable salts) are inhibitors of the enzyme squalene synthase. Thus the compounds of the present invention are capable of inhibiting cholesterol biosynthesis by inhibition of de novo squalene production.

The beneficial pharmacological properties of the compounds of the present invention may be demonstrated using one or more of the following techniques.

### (a) Inhibition of Squalene synthase

In this test, the ability of a compound to prevent the formation of squalene from a radioactive substrate (tritiated farnesyl pyrophosphate) is assessed.

The test compound is incubated at a concentration of 25 micromolar in 200µl of a buffered solution containing potassium phosphate (50mM), MgCl₂ (4.95mM), KF (9.9mM), NADPH (0.9mM) and rat liver microsomal protein (20µg). Rat liver microsomes are prepared by the method described in published European Patent Application No. 324,421 and stored in liquid nitrogen prior to assay. Assay vials are kept at 37°C throughout the incubation.

The reaction is started with the addition of the substrate (1-[³H]-farnesyl pyrophosphate), final concentration 20µM, and stopped after 15 minutes reaction time with the addition of 50µl of 4% KOH. The reaction products are separated from unreacted substrate after application to a C-18 octadecyl lccBond column (Analytichem Int product No. 617101). An aqueous fraction is eluted with 250µl of 0.1M KOH. Squalene is then eluted with 1.0 ml 10% ethylacetate in hexane and radioactivity determined. The difference in radioactivity in the presence and absence of the test compound is used to determine the level of inhibition. If the test compound inhibits at greater than about 70% at 25 micromolar, it is generally re-tested at 25 and 2.5 micromolar. The IC₅₀ (concentration which results in a 50% inhibition of squalene production), of the test compound can be determined by protected alcohol, which may be subsequently oxidised to give the carboxy group. The protecting groups may be removed at any convenient stage in the synthesis using conventional techniques well known in the chemical art.

It will also be appreciated that the preferred process for preparing a particular compound of formula I will depend upon the nature of the various radicals. Similarly, the preferred choice of reagent will depend upon the nature of the various radicals present. For example, when it is required to reduce a particular compound the reducing agent will generally be selected to be one which does not interfere with other groupings present.

It will also be appreciated that certain of the various optional substituents in the compounds of the present invention may be introduced by standard aromatic substitution reactions or generated by conventional functional group modifications either prior to or immediately following the processes mentioned above, and as such are included in the process aspect of the invention. Such reactions and modifications include, for example, introduction of a substituent by means of an aromatic substitution reaction, reduction of substituents, alkylation of substituents and oxidation of substituents. The reagents and reaction conditions for such procedures are well known in the chemical art. Particular examples of aromatic substitution reactions include the introduction of a nitro group using concentrated nitric acid, the introduction of an acyl group using, for example, an acylhalide and Lewis acid (such as aluminium trichloride) under Friedel Crafts conditions; the introduction of an alkyl group using an alkyl halide and Levis acid (such as aluminium trichloride) under Friedel Crafts conditions; and the introduction of a halogeno group. Particular examples of modifications include the reduction of a nitro group to an amino group by for example, catalytic hydrogenation with a nickel catalyst or treatment with iron in the presence of hydrochloric acid with heating; oxidation of alkylthio to alkylsulphinyl or alkylsulphonyl.

When a pharmaceutically-acceptable salt of a compound of the formula I is required, it may be obtained, for example, by reaction of said compound with the appropriate acid (which affords a testing the compound at several, for example five, concentrations predicted from the two concentration results. The IC₅₀ can then be determined from a plot of percentage inhibition against concentration of test compound.

In general, compounds of formula I show significant inhibition in the above test at a concentration in the range of about 0.001 to 25µM.

By way of illustration of the squalene synthase inhibitory properties of the compounds of formula I, the compound described in Example 2 below gave an IC₅₀ of 3 x 10⁻⁸M.

### (b) Acute rat cholesterol synthesis assay.

This is an acute in vivo test in the rat to measure de novo hepatic cholesterol synthesis from exogenously administered ¹⁴C-acetate.

Female rats (35 - 55 g) are housed in reverse lighting conditions (red light from 0200h - 1400h) for a period of about 2 weeks prior to test. Animals are allowed free access to chow and drinking water throughout this period. At test, animals should weigh 125 - 150 g.

Test compounds may be administered by oral gavage, dissolved or suspended in 0.5% polysorbate, or by ip or iv dosing. Control animals receive vehicle alone. After 1 hour the rats are injected ip with 25µCi [2-¹⁴C]-acetate (NEN DUPONT. specific activity, 45-60mCi/mmol NEC-085H, or AMERSHAM specific activity, 50-60mCi/mmol CFA 14) in a volume of 0.25 ml saline (100µCi/ml). After a further hour a blood sample was obtained from the abdominal vena cava under terminal halothane anaesthesia.

1ml of plasma is lyophilised and then saponified in 2ml ethanolic KOH (1 part 33% KOH, 9 parts ethanol) at 75°C for 2 hours. After addition of an equal quantity of water, non-saponifiable lipids are extracted with No 5ml volumes of hexane. The hexane extracts are evaporated to dryness and the residues dissolved in ethanol to determine cholesterol specific radioactivity. ED₅₀ values can be determined in the standard way.

In general, compounds of formula I show activity in the range of about 0.1 to 100 mg/kg.

By way of illustration, the compound described in Example 4 below gave an ED₅₀ of 29mg/kg.

No overt toxicity was detected when compounds of the formula I were administered at several multiples of their minimum inhibitory dose or concentration.

As mentioned above, the compounds of the present invention are squalene synthase inhibitors and hence possess the property of inhibiting cholesterol biosynthesis. Thus the compounds of the present invention will be useful in treating diseases or medical conditions in which an inhibition of squalene synthase is desirable, for example those in which a lowering of the level of cholesterol in blood plasma is desirable. In particular, the compounds of the present invention will be useful in treating hypercholesterolemia and/or ischaemic diseases associated with atheromatous vascular degeneration such as atherosclerosis. The compounds of the present invention will also be useful in treating fungal infections.

Thus according to a further feature of the present invention there is provided a method of inhibiting squalene synthase in a warm-blooded animal (such as man) requiring such treatment, which method comprises administering to said animal an effective amount of a compound of formula I (as herein defined), or a pharmaceutically-acceptable salt thereof. In particular, the present invention provides a method of inhibiting cholesterol biosynthesis, and more particularly to a method of treating hypercholesterolemia and atheromatous vascular degeneration (such as atherosclerosis).

Thus the present invention also provides the use of a compound of formula I (as herein defined), or a pharmaceutically-acceptable salt thereof, for the manufacture of a medicament for treating diseases or medical conditions in which a lowering of the level of cholesterol in blood plasma is desirable (such as hypercholesterolemia and atherosclerosis).

When used in the treatment of diseases and medical conditions in which an inhibition of cholesterol biosynthesis is desired, for example in the treatment of hypercholesterolemia or atherosclerosis, it is envisaged that a compound of formula I (or a pharmaceutically acceptable salt thereof) will be administered orally, intravenously, or by some other medically acceptable route so that a dose in the general range of, for example, 0.01 to 50 mg per kg body weight is received. However it will be understood that the precise dose administered will necessarily vary according to the nature and severity of the disease, the age and sex of the patient being treated and the route of administration.

In general, the compounds of formula I (or a pharmaceutically-acceptable salt thereof) will usually be administered in the form of a pharmaceutical composition, that is together with a pharmaceutically acceptable diluent or carrier, and such a composition is provided as a further feature of the present invention.

A pharmaceutical composition of the present invention may be in a variety of dosage forms. For example, it may be in the form of tablets, capsules, solutions or suspensions for oral administration, in the form of a suppository for rectal administration; in the form of a sterile solution or suspension for parenteral administration such as by intravenous or intramuscular injection.

A composition may be obtained by conventional procedures using pharmaceutically acceptable diluents and carriers well known in the art. Tablets and capsules for oral administration may conveniently be formed with a coating, such as an enteric coating (for example, one based on cellulose acetate phthalate), to minimise dissolution of the active ingredient of formula I (or a pharmaceutically-acceptable salt thereof) in the stomach or to mask unpleasant taste.

The compounds of the present invention may, if desired, be administered together with (or sequentially to) one or more other pharmacological agents known to be useful in the treatment of cardiovascular disease, for example, together with agents such as HMG-CoA reductase inhibitors, bile acid sequestrants, other hypocholesterolaemic agents such as fibrates, for example gemfibrozil, and drugs for the treatment of coronary heart disease. As a further example, the compounds of the present invention may, if desired, be administered together with (or sequentially to) an angiotensin converting enzyme (ACE) inhibitor, such as captopril, lisinopril, zofenopril or enalapril.

The compounds of the present invention may also find utility as antifungal agents, and so the present invention also provides a method of treating fungal infections which comprises administration to a warm blooded animal, such as man, in need of such treatment an effective amount of a compound of formula I, or a pharmaceutically acceptable salt thereof. When used in this way the compounds of the present invention may, in addition to the formulations mentioned above, be adapted for topical administration and such a composition is provided as a further feature of the present invention. Such compositions may be in a variety of forms, for example creams or lotions.

The invention will now be illustrated by the following non-limiting Examples in which, unless otherwise stated:-
(i) evaporations were carried out by rotary evaporation in vacuo;
(ii) operations were carried out at room temperature, that is in the range 18-26°C;
(iii) yields are given for illustration only and are not necessarily the maximum attainable by diligent process development;
(iv) proton NMR spectra were normally determined at 200 MHz using tetramethylsilane (TMs) as an internal standard, and are expressed as chemical shifts (delta values) in parts per million relative to TMs using conventional abbreviations for designation of major peaks: s, singlet; m, multiplet; t, triplet; br, broad; d,doublet;
(v) all end-products were characterised by microanalysis, NMR and/or mass spectroscopy;
(vi) conventional abbreviations are used for individual radicals and recrystallisation solvents, for example, Me = methyl, Et = ethyl, Pr = Propyl, Prⁱ = isopropyl, Bu = butyl, Buⁱ = isobutyl, Ph = phenyl; EtOAc = ethyl acetate, Et₂O = ether, MeCN = acetonitrile, HeOH - methanol, EtOH = ethanol, PrⁱOH = 2-propanol, H₂O = water.

### EXAMPLE 1

A solution of sec-butyl lithium in cyclohexane (3.0 ml, 1.3M) was added dropwise with stirring to a solution of 1-benzyloxy-4-bromo benzene (840 mg) in dry tetrahydrofuran (10 ml) under an argon atmosphere at -78°C. The mixture was stirred for 10 minutes. A solution of quinuclidin-3-one (375 mg) in dry tetrahydrofuran (5 ml) was then added over a period of 10 mmutes. The reaction mixture was stirred at -70°C for 2 hours. The reaction mixture was then stirred for a further period of 16 hours during which time the reaction mixture was allowed to warm to 20°C. The reaction mixture was added to cold (0°C) 1M hydrochloric acid (100 ml). The aqueous solution was washed with diethyl ether (3 x 50 ml) before the addition of sufficient 10M sodium hydroxide solution to give a pH of 14. The-mixture was extracted with ethyl acetate (3 x 50 ml). The ethyl acetate extracts were combined, washed with saturated brine solution (2 x 50 ml), dried (Na₂SO₄) and evaporated to give an oil which was purified on neutral alumina (Type 507 C) using dichlorormethane/methanol/0.88 ammonia 87:12:1 as eluent. Evaporation of product containing fractions gave a foam which gave a colourless solid on trituration with ethyl acetate. This solid was further purified by recrystallisation from ethyl acetate to give 3-(4-benzyloxyphenyl)-quinuclidin-3-ol (140 mg) as a colourless -solid, m.p. 132-133°C; microanalysis, found: C, 77.2; H, 7.8; N, 4.5%; C₂₀H₂₃NO₂ requires C, 77.6; H, 7.5; N, 4.4%; NMR: (CDCl₃) 1.49(3H, m), 2.02(1H, OH), 2.21(2H, m), 2.95(5H, m), 3.47(1H, d of d), 5.08(2H, s), 6.98(2H, m), 7.41(7H, 1). m/Z 310 (M+H)⁺.

The starting 1-benzyloxy-4-bromobenzene was prepared by the method of Powell and Adams, J.A.C.S., 42, 657, (1920).

### EXAMPLE 2

Using a similar procedure to that described in Example 1 but using 1-phenoxymethyl-4-bromobenzene as starting material (in place of 1-benzyloxy 4-bromobenzene) there was obtained 3-(4-phenoxymethylphenyl)-quinuclidin-3-ol (49% yield), m.p. 182-183°C, microanalysis, found: C, 68.6; H, 6.9; N, 4.0%; C₂₀H₂₃NO₂.0.25H₂O requires: C, 68.6; H, 7.0; N, 4.0%; NMR ([CD₃)₂SO/CD₃COOD) 1.6-1.7(1H, m), 1.8-1.9(2H, m), 2.5(2H, s), 3.2-3.4(4H, m), 3.5-4.0(2H, d of d), 5.15(2H, s), 6.9-7.1(3H, m), 7.2-7.3(2H, m) and 7.5-7.7(4H, q).
m/Z 310 (M+H)⁺.

The starting 1-phenoxymethyl-4-bromobenzene was prepared as follows:

A solution of 4-bromobenzylbromide (2.49 g) and phenol (1.13 g) in dry butan-2-one (40 ml) was heated under refulx with potassium carbonate (3.77 g) for 16 hours. After filtration, the solution was evaporated and the residue dissolved in 1M sodium hydroxide solution (50 ml). The aqueous mixture was extracted with diethyl ether (2 X 50 ml), the ether extracts combined, dried (MgSO₄) and evaporated to give 1-phenoxymethyl-4-bromobenzene (2.37 g) as a colourless solid, m.p. 96-97°C.

### EXAMPLE 3

Using a similar procedure to that described in Example 1 but using E-4-bromostilbene as starting material (in place of 1-benzyloxy-4-bromobenzene) there was obtained 3-(E-4-phenylethen-2-ylphenyl)-quinuclidin-3-ol (59% yield), m.p. 190-192°C, microanalysis, found: C, 81.4; H, 7.7; N, 4.6%; C₂₁H₂₃NO.0.25H₂O requires: C, 81.4; H, 7.6; N, 4.5%; NMR ([CD₃)₂SO/CD₃COOD) 1.6-1.7(1H, m), 1.8-1.9(2H, m), 2.4(2H, s), 3.2-3.4(4H, m), 3.5-4.0(2H, d of d), 7.2-7.4(5H, m) and 7.5-7.7(6H, m); m/Z 306 (M+H)⁺.

The starting E-4-bromostilbene was prepared by the method of Wood and Mallory. J.O.C., 29, 3373, (1964).

### EXAMPLE 4

Using a similar procedure to that described in Example 1 but using 4-bromo-diphenylacetylene as starting material (in place of 1-benzyloxy 4-bromobenzene) there was obtained 3-(4-phenylethyn-2-ylphenyl)quinuclidin-3-ol (49% yield), m.p. 218-219°C; microanalysis, found: C, 82.1; H, 7.0; N, 4.6%; C₂₁H₂₁NO 0.2H₂O requires: C, 82.2; H, 7.0; N, 4.6%) NMR (CDCl₃) 1.4-1.6(3H, m), 2.2-2.4(3H, m), 2.7-3.0(4H, m), 3.1-3.6(2H, d of d), and 7.3-7.6(9H, m). m/Z 304 (M+H)⁺.

The starting 4-bromodiphenylacetylene was prepared by the method of Misurri et all Bull. Chem. Soc. Japan, 34, 1833, (1961).

### EXAMPLE 5

A solution of sec-butyllithium in cyclohexane (10 ml, 1.3M) was added dropwise with stirring to a solution of 1-benzyloxy-4-bromobenzene (2.80 g) in dry tetrahydrofuran (25 ml) under an argon atmosphere at -78°C. The mixture was stirred for 15 minutes and a solution of quinuclidin-3-one (1.25 g) in dry tetrahydrofuran (20 ml) was added over a period of 5 minutes. The reaction mixture was stirred at -70°C for 30 minutes and then-allowed to warm to ambient temperature overnight. The reaction mixture was added to cold 1M hydrochloric acid (100 ml). The aqueous solution was washed with diethyl ether (2 x 50 ml) and then basified to- pH 14 using 8M sodium hydroxide solution. The mixture was extracted with ethyl acetate (3 x 50 ml) and the ethyl acetate extracts were combined, washed with saturated brine solution (2 x 50 ml), dried (Na₂SO₄) and evaporated to give an oil. This oil was dissolved in propan-2-ol and ethereal hydrogen chloride added to pH 2. The mixture was heated to reflux and then cooled. Addition of diethyl ether gave a solid which was recrystallised from propan-2-ol to give 3-(4-benzyloxyphenyl)-2,3-dehydroquinuclidine hydrochloride (510 mg) as a solid, m.p. 247-249°C, microanalysis, found: C, 72.8; H, 6.9; N, 4.2%; C₂₀H₂₁NO.HCl requires C, 73.2; H, 6.9; N, 4.2%; NHR: ([CD₃]₂SO/CD₃COOD) 1.63-1.80(2H, m), 1.96-2.15(2H, m), 2.98-3.15(2H, m), 3.50-3.67(3H,m), 5.16(2H, s), 6.92(1H, s), 7.07(2H, d), 7.30-7.50(5H, m) and 7.54(2H, d). m/Z 292 (M+H)⁺.

### EXAMPLE 6

3-(4-Phenylethen-2-ylphenyl)quinuclidin-3-ol (610 mg) in absolute ethanol (30 ml) was hydrogenated at atmospheric pressure for 4 hours over a catalyst of 10% palladium on carbon. The catalyst was removed by filtration and the alcohol evaporated. The residue was crystallised from ethyl acetate to give 3-(4-phenylethylphenyl)-qninuclidin-3-ol (370 mg) as a colourless solid, m.p. 134-135°C, microanalysis, found: C, 81.2; H, 8.1; N, 4.6%; C₂₁H₂₅NO 0.2H₂O requires: C, 81.2; H, 8.2; N, 4.5%; NMR: ([CD₃]₂SO/CD₃COOD) 1.5-1.6(1H, m), 1.8-1.9(2H, m), 2.4(2H, s), 2.9(4H, s), 3.2-3.4(4H, m), 3.4-3.9(2H, d of d) and 7.2-7.5(9H, m).
m/Z 308 (M+H)⁺.

### EXAMPLE 7

Using a similar procedure to that described in Example 1 but using 4-[(Z)-4-methoxystyryl]bromobenzene as starting material (in place of 1-benzyloxy-4-bromobenzene) there was obtained 3-(4-[(Z)-4-methoxystyryl]phenyl)quinuclidin-3-ol (54% yield), m.p. 137-138°C, microanalysis, found: C, 79.0; H, 7.8; N, 3.9%; C₂₂H₂₅NO₂ requires: C,78.8; H, 7.5; N, 4.2%; NMR (CDCl₃): 1.4-1.6(3H,m), 2.2-2.4(3H,m), 2.7-3.0(4H,m), 3.1-3.5(2H, d of d), 3.8(3H,s), 6.4-6.6(2H,q), 6.8(2H,d) and 7.2-7.4(6H,m); m/z 336(M+H).

The 4-[(Z)-4-methoxystyryl]bromobenzene used as starting material was prepared as follows.

4-methoxybenzyltriphenylphosphonium chloride (34.3g) was added to a solution of potassium butoxide (12.0g) in tetrahydrofuran (230ml) over a period of 30 minutes. The reaction was stirred for 1 hour, and a solution of 4-bromobenzaldehyde (14.4g) in tetrahydrofuran (70ml) was then added to the reaction mixture over a period of 10 minutes. The reaction mixture was stirred for 1 hour and water (200ml) was then added to the reaction mixture over a period of 20 minutes. The aqueous mixture was extracted with diethyl ether (2 x 200ml), the ether extracts combined, dried (MgSO₄) and evaporated. The residue was extracted with n-hexane (200ml), filtered and evaporated to give 4-[(Z)-4-methoxystyryl]bromobenzene as a colourless oil, NMR (CDCl₃): 3.8 (3H,s), 6.4-6.6(2H,q), 6.8(2H,d)- and 7.1-7.4(6H,m).

### EXAMPLE 8

Using a similar procedure to that described in Example 1 but using 4-[(E)-3,4-methylenedioxystyryl]bromobenzene as starting material (in place of 1-benzyloxy-4-bromobenzene) there was obtained 3-(4-[(E)-3,4-methylenedioxystyryl]phenyl)quinuclidin-3-ol (52% yield), m.p. 206-207°C, microanalysis, found; C, 75.3% H, 6.6; N, 3.9%; C₂₂H₂₃NO₃ requires: C, 75.6; H, 6.6; N, 4.0%; NMR([CD₃]₂SO/CD₃COOD): 1.5-1.7(1H,m), 1.7-1.9(2H,m), 2.3-2.5(2H,m), 3.1-3.4(4H,m), 3.4-3.9(2H, d of d), 6.0(2H,s), 6.9(1H,d), 7.1(1H, d of d), 7.15(2H,d), 7.25(1H,d), and 7.6(4H,s); m/z 350 (M+H).

The 4-[(E)-3,4-methylenedioxystyryl]bromobenzene used as starting material was prepared as follows.

4-Bromobenzyltriphenylphosphonium bromide (5.0g) was added to a solution of potassium butoxide (1.34g) in tetrahydrofuran (50ml) over a period of 5 minutes. The reaction mixture was stirred for 1 hour and a solution of piperonal (1.56g) in tetrahydrofuran was then added to the reaction mixture over a period of 10 minutes. The reaction mixture was stirred for a further 1 hour. Water (70ml) was added to the reaction mixture over a period of 10 minutes and the aqueous mixture was extracted with diethyl ether (2 x 70ml). The ether extracts were combined, dried (MgSO₄) and evaporated. The residue was crystallised from n-hexane to give 4-[(E)-3,4-methylenedioxystyryl]bromobenzene as a colourless solid, m.p. 139-140°C, microanalysis, found: C, 59.2; H, 3.5%; C₁₅H₁₁BrO₂ requires: C, 59.4; H,3.7%; NMR (CDCl₃): 6.0(2H,s), 6.8(1H,s), 6.9(3H,m), 7.1(1H,s) and 7.3-7.5(4H, d of d); m/z 302(M).

### EXAMPLE 9

Using a similar procedure to that described in Example 1 but using 4-[(E)-4-tertbutyldimethylsilyloxystyryl]bromobenzene as starting material (in place of 1 benzyloxy-4-bromobenzene) there was obtained 3-(4-[(E)-4-hydroxystyryl]phenyl)quinuclidin-3-ol (21% yield), m.p. 260-261°C, microanalysis, found: C, 77.3; H, 7.2; N, 4.0%; C₂₁H₂₃NO₂ 0.33H₂O requires: C, 77.1; H, 7.2; N, 4.3%; NMR ([CD₃]₂SO/CD₃COOD); 1.3-1.5(1H,m), 1.6-1.7(2H,m), 2.2-2.4(2H,m), 3.0-3.2 (4H,m), 3.3-3.8(2H, d of d), 6.7(2H,d), 6.9-7.1(2H,q), 7.3(2H,d) and 7.4(4H,s); m/z 322 (M+H).

The 4-[(E)-4-tertbutyldimethylsilyloxystyryl] bromobenzene used as starting material was prepared in a similar method to 4-[(E)-3,4-methylenedioxystyryl]bromobenzene described in Example 8, m.p. 136-138°C, microanalysis, found: C, 61.9; H, 6.5%; C₂₀H₂₅BrOSi requires: C,61.7; H, 6.5%; NMR(CDCl₃): 0.2(6H,s), 1.0(9H,s), 6.8(2H,d), 6.8-7.1(2H,q), and 7.3-7.5(6H,m); m/z 389(M+H).

### EXAMPLE 10

A 1.0M solution of boron tribromide in dichloromethane (10ml) was added at -70°C to a solution of 3-(4-[(E)-4-methoxystyryl]-phenyl)quinuclidin-3-ol (825mg) in dichloromethane (125ml) over a period of 20 minutes. The reaction mixture was stirred for 2 hours at -70°C and then for 12 hours at room temperature. A saturated solution of sodium hydrogen carbonate (45ml) was then added to the reaction mixture over a period of 20 minutes. The solid precipitate was collected by filtration, washed with water and dried. The solid was dissolved in methanol (50ml), and an excess of saturated ethereal hydrogen chloride solution added. The resulting solution was evaporated and the residue crystallised from a mixture of methanol and ethyl acetate to give 3-(4-[(E)-4-hydroxystyryl]phenyl)-2,3-dehydroquinuclidine hydrochloride as a colourless solid (10% yield), m.p.274-275°C, microanalysis, found: C, 69.7; H, 6.2; N, 3.6%; C₂₁H₂₁NO.HCl.1.25 H₂O requires: C, 69.6; H, 6.7; N, 3.9%. NMR ([CD₃]₂SO): 1.6-1.8(2H,m), 1.9-2.1(2H,m), 2.9-3.1(2H,m), 3.5-3.6(3H,m), 6.8(2H,d), 7-0-7.3(2H,q), 7.1(1H,s), 7.4(2H,d) and 7.6(4H,s); m/z 304 (M+H).

### EXAMPLE 11

Using a similar procedure to that described in Example 1 but using N-(4-bromophenyl)benzamide as starting material (in place of 1-benzyloxy-4-bromobenzene) and using 2.2 molar equivalens of sec-butyl lithium, there was obtained 3-(4-benzoylaminophenyl)quinuclidinin-3-ol (18% yield), m.p. 226-227°C, microanalysis, found: C, 74.6; H, 7.1; N, 8.6%: C₂₀H₂₂N₂O₂ requires: C, 74.5; H, 6.9; N, 8.7%; NHR ([CD₃]₂SO): 1.2-1.5(3H,m), 1.9(1H,s), 2.0-2.2(1H,m), 2.6-2.8(4H,m), 2.9-3.4(2H, d of d), 5,0(1H,s), 7.4-7.6(5H,m), 7.7-7.8(2H;d), 8.0(2H, d of-d) and 10.2 (1H,s) ; m/z 323(M+H).

### EXAMPLE 12

Illustrative pharmaceutical dosage forms suitable for presenting the compounds of the invention for therapeutic or prophylactic use include the following tablet and capsule formulations, which may be obtained by conventional procedures well known in the art of pharmacy and are suitable for therapeutic or prophylactic use in humans:-

| (a) **Tablet I** | |
|---|---|
| | **mg/tablet** |
| Compound Z* | 1.0 |
| Lactose Ph. Eur. | 93.25 |
| Croscarmellose sodium | 4.0 |
| Maize starch paste (5% w/v aqueous paste) | 0.75 |
| Magnesium stearate | 1.0 |

| (b) **Tablet II** | **mg/tablet** |
|---|---|
| Compound Z* | 50 |
| Lactose Ph. Eur | 223.75 |
| Croscarmellose sodium | 6.0 |
| Maize starch | 15.0 |
| Polyvinylpyrrolidone (5% w/v aqueous paste) | 2.25 |
| Magnesium stearate | 3.0 |

| (c) **Tablet III** | **mg/tablet** |
|---|---|
| Compound Z* | 100 |
| Lactose Ph. Eur. | 182.75 |
| Croscarmellose sodium | 12.0 |
| Maize starch paste (5% w/v aqueous paste) | 2.25 |
| Magnesium stearate | 3.0 |

| (d) **Capsule** | |
|---|---|
| | **mg/capsule** |
| Compound Z* | 10 |
| Lactose Ph.Eur. | 488.5 |
| Magnesium stearate | 1.5 |

| | |
|---|---|
| Note * The active ingredient Compound Z is a compound of formula I, or a salt thereof, for example a compound of formula I described in any of the preceding Examples. | |

The tablet compostions (a) - (c) may be enteric coated by conventional means, for example, with cellulose acetate phthalate.

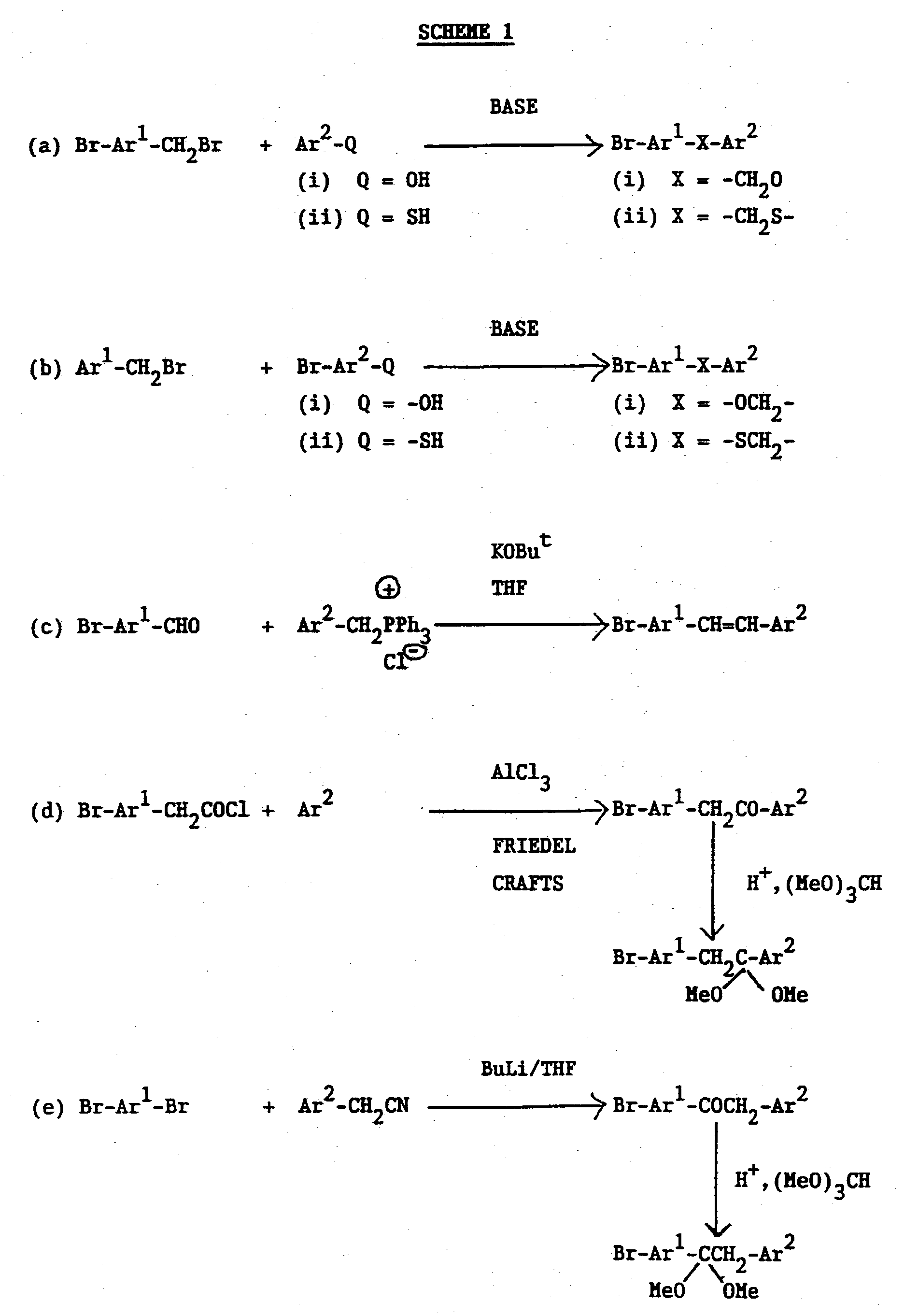

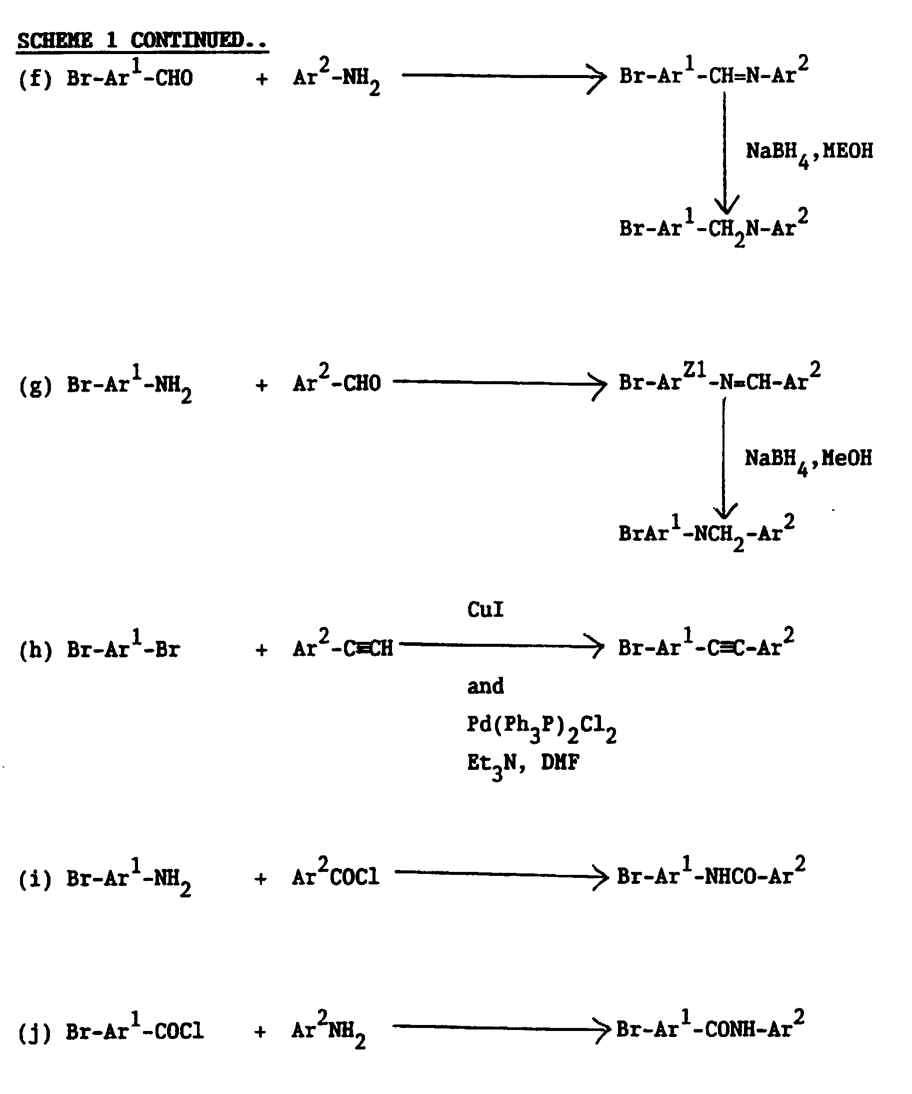

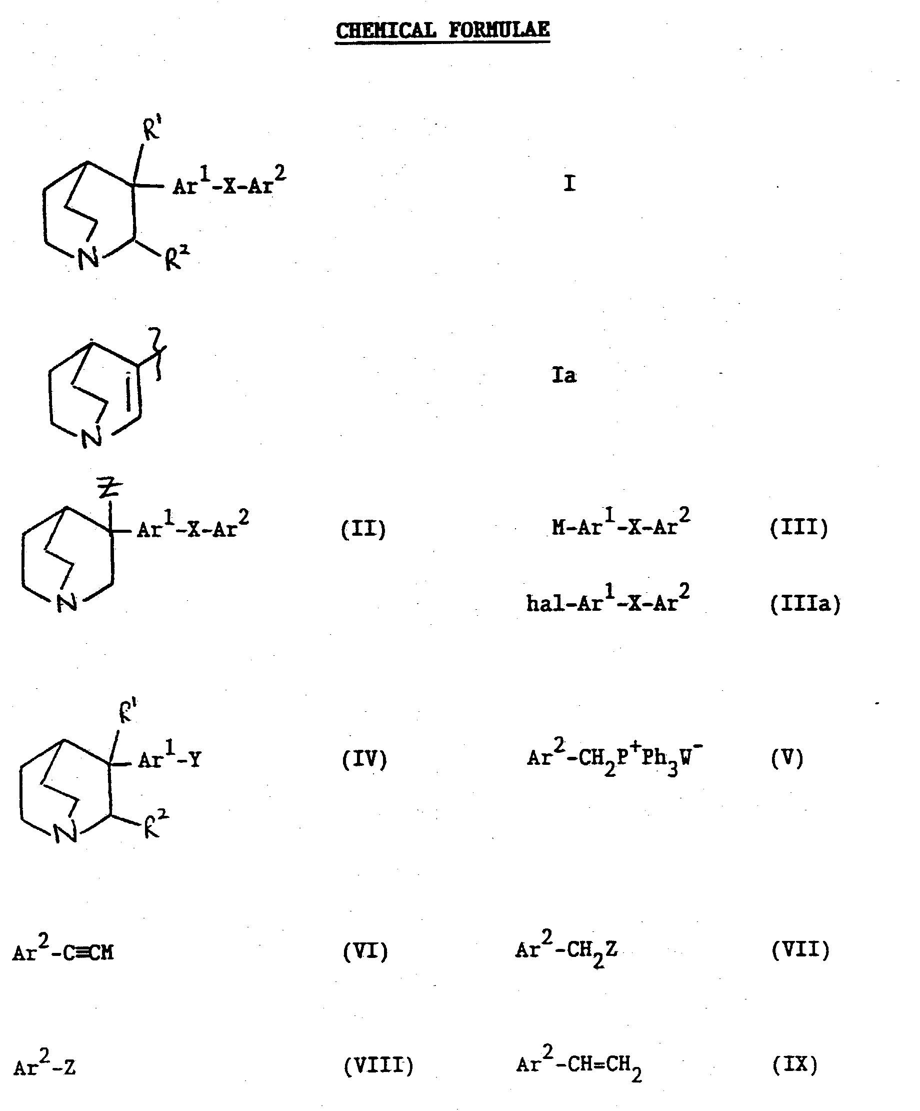

## Claims

1. A compound of formula I, or a pharmaceutically acceptable salt thereof, 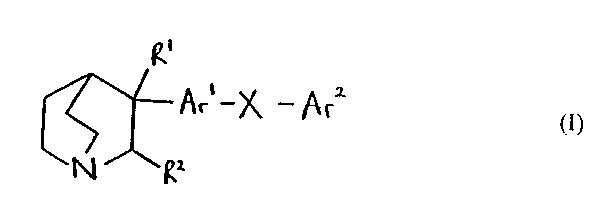 wherein:
R¹ is hydrogen or hydroxy;
R² is hydrogen;
or R¹ and R² are joined together so that CR¹-CR² is a double bond;
X is selected from -CH₂CH₂-, -CH=CH-, -C≡C-, -CH₂O-, -OCH₂-, -CH₂NH-, -NHCH₂-, -CH₂CO-, -COCH₂-, -CH₂S-, -SCH₂-, wherein the sulphur atom in the latter two groups may optionally bear one or two oxygen atoms;
Ar¹ is a 1,4-phenylene moiety;
Ar² is phenyl; and
wherein one or both of Ar¹ and Ar² may be optionally unsubstituted or independently substituted by one or more substituents selected from halogeno, hydroxy, amino, nitro, cyano, carboxy, carbamoyl, (1-6C)alkyl, (2-6C)alkenyl, (1-6C)alkoxy, (1-6C)alkylamino, di-[(1-6C)alkyl]amino, N-[(1-6C)alkyl]carbamoyl, N,N-di-[(1-6C)alkyl]carbamoyl, (1-6C)alkoxycarbonyl, (1-6C)alkylthio, (1-6C)alkylsulphinyl, (1-6C)alkylsulphonyl, halogeno-(1-6C)alkyl, and (1-6C)alkanoylamino.

2. A compound as claimed in claim 1 wherein R¹ is hydrogen or hydroxy; R² is hydrogen; or R¹ and R² are joined together so that CR¹-CR² is a double bond;
X is selected from -CH₂CH₂-, -CH=CH-, -C≡C-, -CH₂O-, -OCH₂-, -CH₂NH-, -NHCH₂-, -CH₂CO-, -COCH₂-, -CH₂S-, -SCH₂-, wherein the sulphur atom in the latter two groups may optionally bear one or two oxygen atoms;
Ar¹ is a 1,4-phenylene moiety;
Ar² is phenyl; and
wherein one or both of Ar¹ and Ar² may be optionally unsubstituted or independently substituted by one or more substituents selected from hydroxy, amino, nitro, cyano, carboxy, carbamoyl, methylenedioxy, isopropylidenedioxy, fluoro, chloro, bromo, methyl, ethyl, propyl, isopropyl, butyl, isobutyl, sec-butyl, allyl, but-2-enyl, 2-methyl-2-propenyl, methoxy, ethoxy, propoxy, isopropoxy, butoxy, methylamino, ethylamino, propylamino, butylamino, dimethylamino, diethylamino, methylpropylamino, dipropylamino, N-methylcarbamoyl, N-ethylcarbamoyl, N-propylcarbamoyl, N,N-dimethylcarbamoyl, N,N-diethylcarbamoyl, methoxycarbonyl, ethoxycarbonyl, propoxycarbonyl, methylthio, ethylthio, propylthio, isopropylthio, butylthio, methylsulphinyl, ethylsulphinyl, propylsulphinyl, isopropylsulphinyl, butylsulphinyl, methylsulphonyl, ethylsulphonyl, propylsulphonyl, isopropylsulphonyl, butylsulphonyl, fluoromethyl, difluoromethyl, trifluoromethyl, butyryl, formamido, acetamido and propionamido.

3. A compound as claimed in claim 1 or 2 wherein R¹ is hydroxy and R² is hydrogen.

4. A compound as claimed in any one of the preceding claims wherein X is selected from -CH₂CH₂-, -CH=CH-, -C≡C-, -CH₂O-, and -OCH₂-.

5. A compound as claimed in claim 1 selected from:
3-(4-benzyloxyphenyl)quinuclidin-3-ol;
3-(4-phenoxymethylphenyl)quinuclidin-3-ol;
3-(E-4-phenylethen-2-ylphenyl)quinuclidin-3-ol;
3-(4-phenylethyn-2-ylphenyl)quinuclidin-3-ol;
3-(4-benzyloxyphenyl)-2,3-dehydroquinuclidine;
3-(4-phenylethylphenyl)quinuclidin-3-ol;
and pharmaceutically acceptable salts thereof.

6. A compound of formula I, or a pharmaceutically acceptable salt thereof, 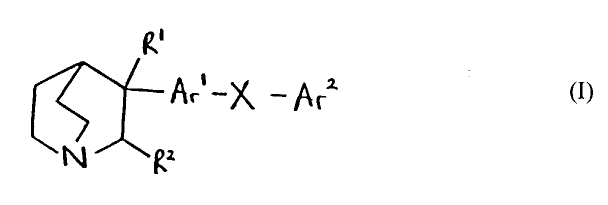 wherein:
R¹ is hydroxy;
R² is hydrogen;
X is selected from -CH₂CH₂-, -CH=CH-, -C≡C-, -CH₂O-, -OCH₂-, -CH₂NH-, -NHCH₂-, -CH₂CO-, -COCH₂-, -NHCO-, -CONH-, -CH₂S-, -SCH₂-, wherein the sulphur atom in the latter two groups may optionally bear one or two oxygen atoms;
Ar¹ is a phenylene moiety;
Ar² is phenyl; and
wherein one or both of Ar¹ and Ar² may be optionally unsubstituted or independently substituted by one or more substituents selected from halogeno, hydroxy, amino, nitro, cyano, carboxy, carbamoyl, (1-6C)alkyl, (2-6C)alkenyl, (2-6C)alkynyl, (1-6C)alkoxy, (1-6C)alkylamino, di-[(1-6C)alkyl]amino, N-[(1-6C)alkyl]carbamoyl, N,N-di-[(1-6C)alkyl]carbamoyl, (1-6C)alkoxycarbonyl, (1-6C)alkylthio, (1-6C)alkylsulphinyl, (1-6C)alkylsulphonyl, halogeno-(1-6C)alkyl, (1-6C)alkanoyl, (1-4C)alkylenedioxy and (1-6C)alkanoylamino.

7. A compound as claimed in claim 6 wherein R¹ is hydrogen or hydroxy; R² is hydrogen;
X is selected from -CH₂CH₂-, -CH=CH-, -C≡C-, -CH₂O-, -OCH₂-, -CH₂NH-, -NHCH₂-, -CH₂CO-, -COCH₂-, -NHCO-, -CONH-, -CH₂S-, -SCH₂-, wherein the sulphur atom in the latter two groups may optionally bear one or two oxygen atoms:
Ar¹ is a phenylene moiety;
Ar² is phenyl; and
wherein one or both of Ar¹ and Ar² may be optionally unsubstituted or independently substituted by one or more substituents selected from hydroxy, amino, nitro, cyano, carboxy, carbamoyl, methylenedioxy, isopropylidenedioxy, fluoro, chloro, bromo, methyl, ethyl, propyl, isopropyl, butyl, isobutyl, sec-butyl, allyl, but-2-enyl, 2-methyl-2-propenyl, prop-2-ynyl, but-2-ynyl, methoxy, ethoxy, propoxy, isopropoxy, butoxy, methylamino, ethylamino, propylamino, butylamino, dimethylamino, diethylamino, methylpropylamino, dipropylamino, N-methylcarbamoyl, N-ethylcarbamoyl, N-propylcarbamoyl, N,N-dimethylcarbamoyl, N,N-diethylcarbamoyl, methoxycarbonyl, ethoxycarbonyl, propoxycarbonyl, methylthio, ethylthio, propylthio, isopropylthio, butylthio, methylsulphinyl, ethylsulphinyl, propylsulphinyl, isopropylsulphinyl, butylsulphinyl, methylsulphonyl, ethylsulphonyl, propylsulphonyl, isopropylsulphonyl, butylsulphonyl, fluoromethyl, difluoromethyl, trifluoromethyl, formyl, acetyl, propionyl, butyryl, formamido, acetamido and propionamido.

8. A compound as claimed in claim 6 or 7 wherein Ar¹ comprises a 1,4-phenylene moiety.

9. A compound as claimed in claim 6, 7 or 8 wherein X is selected from -CH₂CH₂-, -CH=CH-, -C≡C-, -CH₂O-, -OCH₂- and -NHCO-.

10. A compound as claimed in claim 6,7, 8 or 9 wherein Art is an unsubstituted 1,4-phenylene moiety and Ar² is phenyl optionally substituted by a (1-6C)alkoxy, hydroxy or (1-4C)alkylenedioxy group.

11. A compound as claimed in claim 6 selected from:
3-(4-[(Z)-4-methoxystyryl]phenyl)quinuclidin-3-ol;
3-(4-[(E)-3,4-methylenedioxystyryl]phenyl)quinuclidin-3-ol,
3-(4-[(E)-4-hydroxystyryl]phenyl)quinuclidin-3-ol; and
3-(4-benzoylaminophenyl)quinuclidin-3-ol;
and pharmaceutically acceptable salts thereof.

12. A process for the preparation of a compound of formula I, or a pharmaceutically acceptable salt thereof, as claimed in claim 1 or 6, which process is selected from:
(a) for those compounds of formula I in which R¹ and R² are both hydrogen, reducing a compound of formula I in which R¹ and R² are joined together so that CR¹-CR² is a double bond;
(b) for compounds of formula I in which R¹ and R² are joined together so that CR¹-CR² is a double bond, dehydrating a compound of formula I in which R¹ is hydroxy and R² is hydrogen;
(c) for compounds of formula I in which R¹ and R² are joined together so that CR¹-CR² is a double bond, treating a compound of formula II 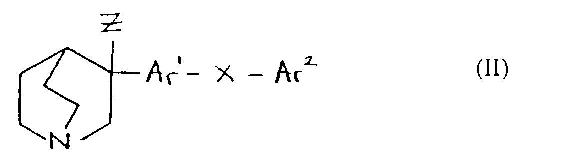 in which Z is a leaving group with a base;
(d) for those compounds of formula I in which R¹ is hydroxy and R² is hydrogen, reacting an organometallic derivative of formula III :-
M-Ar¹⁻X-Ar² (III)
in which M is a metal atom or a derivative thereof, with quinuclidin-3-one;
(e) for those compounds of formula I in which X is -CH=CH-, reacting a compound of formula IV: 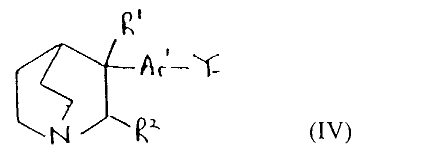 with a compound of formula V:
Ar²-CH₂P⁺Ph₃W- (V)
in which W is a halogen atom, in the presence of a base;
(f) for those compounds of formula I in which X is -C≡C-, reacting a compound of formula IV: 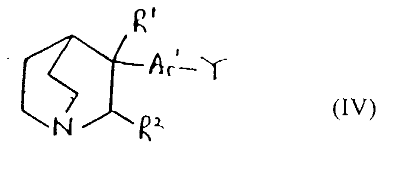 in which Y is a leaving group with a compound of formula VI:
Ar²-C≡CM (VI)
in the presence of a catalyst;
(g) for those compounds of formula I in which X is -OCH₂-, -NHCH₂- or -SCH₂- reacting a compound of formula IV: 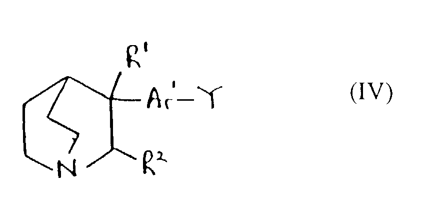 in which Y is -OH, -NH₂ or -SH with a compound of formula VII:
Ar²⁻CH₂Z (VII)
in which Z is a leaving group in the presence of a base;
(h) for those compounds of formula I in which X is -CH₂O-, -CH₂NH- or -CH₂S-, by the reaction a compound of formula IV: 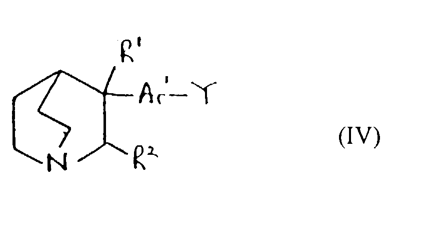 in which Y is -CH₂OH, -CH₂NH₂ or -CH₂SH with a compound of formula Ar²⁻Z in which Z is a leaving group, in the presence of a base; and
(i) for those compound in which X is -CH₂CH₂-, reducing a compound of formula I in which X is -C≡C- or -CH=CH-;
(j) for those compounds of formula I in which X is -CH=CH-, reacting a compound of formula IV: 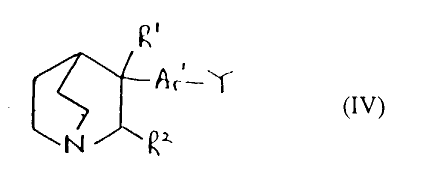 in which Y is a leaving group, with a compound of formula IX:
Ar²⁻CH=CH₂ (IX)
in the presence of a catalyst;
and whereafter, when a pharmaceutically acceptable salt is required, reacting the compound of formula I with an acid which affords a physiologically acceptable anion or a base which affords a physiologically cation (as appropriate).

13. A pharmaceutical composition which comprises a compound of formula I, or a pharmaceutically acceptable salt thereof, as claimed in any one of claims 1 to 11 together with a pharmaceutically acceptable diluent or carrier.

14. The use of a compound of formula I, or a pharmaceutically acceptable salt thereof, as claimed in any one of claims 1 to 11 for the manufacture of a medicament for the treatment of diseases and medical conditions in which an inhibition of cholesterol biosynthesis is desirable.

15. The use as claimed in claim 14 for the manufacture of a medicament for treating hypercholesterolemia or atherosclerosis.

## Patentansprüche

1. Verbindung der Formel I oder ein pharmazeutisch verträgliches Salz davon 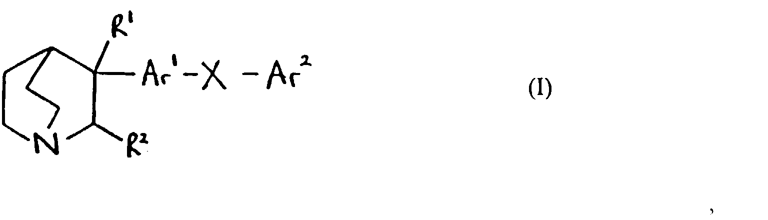 in der
R¹ ein Wasserstoffatom oder eine Hydroxylgruppe ist,
R² ein Wasserstoffatom ist,
oder R¹ und R² so miteinander verbunden sind, daß der Rest CR¹-CR² eine Doppelbindung ist, X ausgewählt ist aus den Gruppen -CH₂CH₂-, -CH=CH-, -C≡C-, -CH₂O-, -OCH₂-_{,} -CH₂NH-, -NHCH₂-_{,} -CH₂CO-, -COCH₂-_{,} -CH₂S- und -SCH₂-, wobei das Schwefelatom in den zwei letztgenannten Gruppen gegebenenfalls ein oder zwei Sauerstoffatome tragen kann,
Ar¹ eine 1,4-Phenyleneinheit ist,
Ar² eine Phenylgruppe ist, und
In der einer der Reste oder beide Reste Ar¹ und Ar² gegebenenfalls unsubstituiert oder unabhängig voneinander substituiert sein können mit einem oder mehreren Substituenten, ausgewählt aus einem Halogenatom, einer Hydroxyl-, Amino-, Nitro-, Cyano-, Carboxy- und Carbamoylgruppe, und einem (1-6C)Alkyl-, (2-6C)Alkenyl-, (1-6C)Alkoxy-, (1-6C)Alkylamino-, Di-[(1-6C)alkyl]amino-, N-[(1-6C)Alkyl]carbamoyl-, N,N-Di-[(1-6C)alkyl]-carbamoyl-, -(1-6C)Alkoxycarbonyl-, (1-6C)Alkylthio-, (1-6C)Alkylsulfinyl-, (1-6C)Alkylsulfonyl-, Halogen-(1-6C)alkyl- und (1-6C)Alkanoylaminorest.

2. Verbindung nach Anspruch 1, in der R¹ ein Wasserstoffatom oder eine Hydroxylgruppe ist,
R² ein Wasserstoffatom ist,
oder R¹ und R² so miteinander verbunden sind, daß der Rest CR¹-CR² eine Doppelbindung ist,
X ausgewählt ist aus den Gruppen -CH₂CH₂-, -CH=CH-, -C≡C-, -CH₂O-, -OCH₂-, -CH₂NH-, -NHCH₂-, -CH₂CO-, -COCH₂-, -CH₂S-, -SCH₂-, wobei das Schwefelatom in den zwei letztgenannten Gruppen gegebenenfalls an ein oder zwei Sauerstoffatome tragen kann,
Ar¹ eine 1,4-Phenyleneinheit ist,
Ar² eine Phenylgruppe ist, und
In der einer der Reste oder beide Reste Ar¹ und Ar² gegebenenfalls unsubstituiert oder unabhängig voneinander substituiert sein können mit einem oder mehreren Substituenten, ausgewählt aus einer Hydroxyl-, Amino-, Nitro-, Cyano-, Carboxy-, Carbamoyl-, Methylen-dioxy- und Isopropylidendioxygruppe, einem Fluor-, Chlor- und Bromatom und einer Methyl-, Ethyl-, Propyl-, Isopropyl-, Butyl-, Isobutyl-, sek-Butyl-, Allyl-, But-2-enyl-, 2-Methyl-2-propenyl-, Methoxy, Ethoxy-, Propoxy, Isopropoxy-, Butoxy-, Methylamino-, Ethylamino-, Propylamino-, Butylamino-, Dimethylamino-, Diethylamino-, Methylpropylamino-, Dipropylamino-, N-Methylcarbamoyl-, N-Ethylcarbamoyl-, N-Propylcarbamoyl-, N,N-Dimethylcarbamoyl-, N,N-Diethylcarbamoyl-, Methoxycarbonyl-, Ethoxycarbonyl-, Propoxycarbonyl-, Methylthio-, Ethylthio-, Propylthio-, Isopropylthio-, Butylthio-, Methylsulfinyl-, Ethylsulfinyl-, Propylsulfinyl-, Isopropylsulfinyl-, Butylsulfinyl-, Methylsulfonyl-, Ethylsulfonyl-, Propylsulfonyl-, Isopropylsulfonyl-, Butylsulfonyl-, Fluormethyl-, Difluormethyl-, Trifluormethyl-, Butyryl-, Formamido-, Acetamido- und Propionamidogruppe.

3. Verbindung nach Anspruch 1 oder 2, in der R¹ eine Hydroxylgruppe ist und R² ein Wasserstoffatom ist.

4. Verbindung nach einem der vorhergehenden Ansprüche, in der X ausgewählt ist aus den Gruppen -CH₂CH₂-, -CH=CH-, -C≡C-, -CH₂O- und -OCH₂-.

5. Verbindung nach Anspruch 1, ausgewählt aus
3-(4-Benzyloxyphenyl)chinuclidin-3-ol,
3-(4-Phenoxymethylphenyl)chinuclidin-3-ol,
3 -(E-4-Phenylethen-2-ylphenyl)chinuclidin-3 -ol,
3-(4-Phenylethin-2-ylphenyl)chinuclidin-3-ol,
3-(4-Benzyloxyphenyl)-2,3-dehydrochinuclidin und
3 -(4-Phenylethylphenyl)chinuclidin-3-ol
und pharmazeutisch verträglichen Salzen davon.

6. Verbindundung der Formel I oder ein pharmazeutisch verträgliches Salz davon 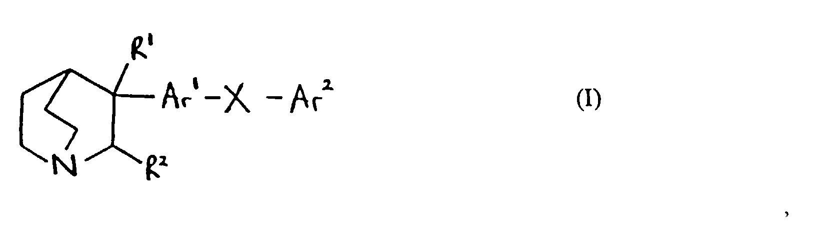 in der
R¹ eine Hydroxylgruppe ist,
R² ein Wasserstoffatom ist,
X ausgewählt ist aus den Gruppen -CH₂CH₂-, -CH=CH-, -C≡C-, -CH₂O-, -OCH₂-, -CH₂NH-, -NHCH₂-, -CH₂CO-, -COCH₂-, -NHCO-, -CONH-, -CH₂S-, -SCH₂-, wobei das Schwefelatom in den zwei letztgenannten Gruppen gegebenenfalls ein oder zwei Sauerstoffatome tragen kann,
Ar¹ eine 1,4-Phenyleneinheit ist,
Ar² eine Phenylgruppe ist, und
In der einer der Reste oder beide Reste Ar' und Ar² gegebenenfalls unsubstituiert oder unabhängig voneinander substituiert sein können mit einem oder mehreren Substituenten ausgewählt aus einem Halogenatom, einer Hydroxyl-, Amino-, Nitro-, Cyano-, Carboxy- und Carbamoylgruppe und einem (1-6C)Alkyl-, (2-6C)Alkenyl-, (2-6C)Alkinyl-, (1-6C)Alkoxy-, (1-6C)Alkylamino-, Di-[(1-6C)alkyl]amino-, N-[(1-6C)Alkyl]carbamoyl-, N,N-Di-[(1-6C)alkyl]carbamoyl-, (1-6C)Alkoxycarbonyl-, (1-6C)Alkylthio-, (1-6C)Alkylsulfinyl-, (1-6C)Alkylsulfonyl-, Halogen-(1-6C)alkyl-, (1-6C)Alkanoyl-, (1-4C)Alkylendioxy- und (1-6C)Alkanoylaminorest.

7. Verbindung nach Anspruch 6, in der R¹ ein Wasserstoffatom oder eine Hydroxylgruppe ist, R² ein Wasserstoffatom ist,
X ausgewählt ist aus den Gruppen -CH₂CH₂-, -CH=CH-, -C≡C-, -CH₂O-, -OCH_{2-,} -CH₂NH-, -NHCH₂-, -CH₂CO-, -COCH₂-, -NHCO-, -CONH-, -CH₂S-, -SCH₂-, wobei das Schwefelatom in den zwei letztgenannten Gruppen gegebenenfalls ein oder zwei Sauerstoffatome tragen kann,
Ar¹ eine Phenyleneinheit ist,
Ar² eine Phenylgruppe ist, und
In der einer der Reste oder beide Reste Ar¹ und Ar² gegebenenfalls unsubstituiert oder unabhängig voneinander substituiert sein können mit einem oder mehreren Substituenten, ausgewählt aus einer Hydroxyl-, Amino-, Nitro-, Cyano-, Carboxy-, Carbamoyl-, Methylen-dioxy- und Isopropylidendioxygruppe, einem Fluor-, Chlor- und Bromatom und einer Methyl-, Ethyl-, Propyl-, Isopropyl-, Butyl-, Isobutyl-, sek-Butyl-, Allyl-, But-2-enyl-, 2-Methyl-2-propenyl-, Prop-2-inyl-, But-2-inyl-, Methoxy, Ethoxy-, Propoxy, Isopropoxy-, Butoxy-, Methylamino-, Ethylamino-, Propylamino-, Butylamino-, Dimethylamino-, Diethylamino-, Methylpropylamino-, Dipropylamino-, N-Methylcarbamoyl-, N-Ethyl-carbamoyl-, N-Propyl-carbamoyl-, N,N-Dimethylcarbamoyl-, N,N-Diethylcarbamoyl-, Methoxycarbonyl-, Ethoxycarbonyl-, Propoxycarbonyl-, Methylthio-, Ethylthio-, Propylthio-, Isopropylthio-, Butylthio-, Methylsulfinyl-, Ethylsulfinyl-, Propyl sulfinyl-, Isopropylsulfinyl-, Butylsulfinyl-, Methylsulfonyl-, Ethylsulfonyl-, Propylsulfonyl-, Isopropylsulfonyl-, Butylsulfonyl-, Fluormethyl-, Difluormethyl-, Trifluormethyl-, Formyl-, Acetyl-, Propionyl-, Butyryl-, Formamido-, Acetamido- und Propionamidogruppe.

8. Verbindung nach Anspruch 6 oder 7, in der Ar¹ eine 1,4-Phenyleneinheit umfaßt.

9. Verbindung nach einem der Ansprüche 6, 7 oder 8, in der X ausgewählt ist aus den Gruppen -CH₂CH₂-, -CH=CH-, -C≡C-, -CH₂O-, -OCH₂- und -NHCO-.

10. Verbindung nach einem der Ansprüche 6, 7, 8 oder 9, in deri Ar¹ eine unsubstituierte 1,4-Phenyleneinheit ist und Ar² eine Phenylgruppe ist, die gegebenenfalls substituiert ist mit einem (1-6C)Alkoxyrest, einer Hydroxylgruppe oder einem (1-4C)Alkylendioxyrest.

11. Verbindung nach Anspruch 6, ausgewählt aus
3-(4-[(Z)-4-Methoxystyryl]phenyl)chinuclidin-3-ol,
3-(4-[(E)-3,4-Methylendioxystyryl]phenyl)chinuclidin-3-ol,
3-(4-[(E)-4-Hydroxystyryl]phenyl)chinuclidin-3-ol und
3 -(4-Benzoylaminophenyl)chinuclidin-3-ol
und pharmazeutisch verträglichen Salzen davon.

12. Verfahren zur Herstellung einer Verbindung der Formel I oder eines pharmazeutisch verträglichen Salzes davon nach Anspruch 1 oder 6, wobei das Verfahren ausgewählt ist aus:
(a) für solche Verbindungen der Formel I, in denen sowohl R¹ als auch R² ein Wasserstoffatom sind, Reduzieren einer Verbindung der Formel I, in der R¹ und R² so miteinander verbunden sind, daß CR¹-CR² eine Doppelbindung ist;
(b) für Verbindungen der Formel I, in denen R¹ und R² so miteinander verbunden sind, daß CR¹-CR² eine Doppelbindung ist, Dehydratisieren einer Verbindung der Formel I, in der R¹ eine Hydroxylgruppe ist und R² ein Wasserstoffatom ist;
(c) für Verbindungen der Formel I, in denen R¹ und R² so miteinander verbunden sind, daß CR¹-CR² eine Doppelbindung ist, Behandeln einer Verbindung der Formel II 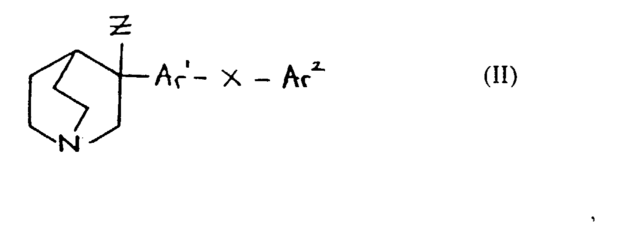 in der Z eine Abgangsgruppe ist, mit einer Base;
(d) für solche Verbindungen der Formel I, in denen R¹ eine Hydroxylgruppe ist und R² ein Wasserstoffatom ist, Umsetzen eines organometallischen Derivats der Formel III
M-Ar¹-X-Ar² (III),
in der M ein Metallatom oder ein Derivat davon ist, mit Chinuclidin-3-on;
(e) für solche Verbindungen der Formel I, in denen X eine Gruppe -CH=CH- ist, Umsetzen einer Verbindung der Formel IV 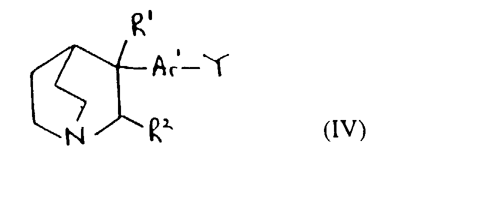 mit einer Verbindung der Formel V
Ar²-CH₂P⁺Ph₃W- (V),
in der W ein Halogenatom ist, in Gegenwart einer Base;
(f) für solche Verbindungen der Formel I, in denen X eine Gruppe -C≡C- ist, Umsetzen einer Verbindung der Formel IV 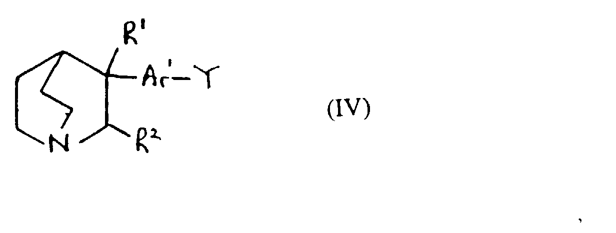 in der Y eine Abgangsgruppe ist, mit einer Verbindung der Formel VI
Ar²-C≡CM (VI)
in Gegenwart eines Katalysators;
(g) für solche Verbindungen der Formel I, in denen X eine Gruppe -OCH₂-, NHCH₂- oder SCH₂- ist, Umsetzen einer Verbindung der Formel IV 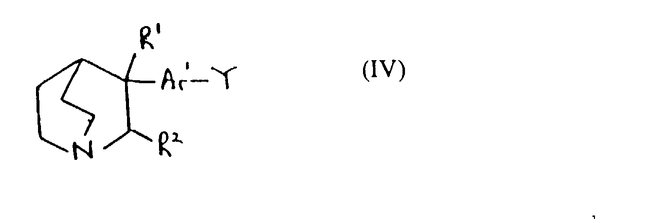 in der Y eine Gruppe -OH, -NH₂ oder -SH ist, mit einer Verbindung der Formel VII
Ar²CH₂Z (VII),
in der Z eine Abgangsgruppe ist, in Gegenwart einer Base;
(h) für solche Verbindungen der Formel I, in denen X eine Gruppe -CH₂O-, -CH₂NH- oder -CH₂S- ist, durch Umsetzen einer Verbindung der Formel IV 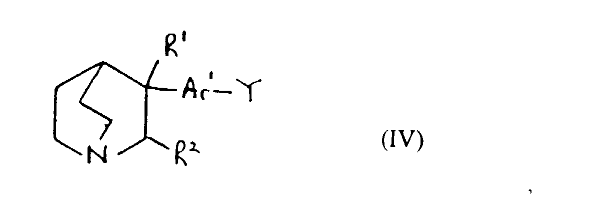 in der Y eine Gruppe -CH₂OH, -CH₂NH₂ oder -CH₂SH ist, mit einer Verbindung der Formel Ar²-Z, in der Z eine Abgangsgruppe ist, in Gegenwart einer Base; und
(i) für solche Verbindungen, in denen X eine Gruppe -CH₂CH₂- ist, Reduzieren einer Verbindung der Formel I, in der X eine Gruppe -C≡C- oder -CH=CH- ist;
(j) für solche Verbindungen der Formel I, in denen X eine Gruppe -CH=CH- ist, Umsetzen einer Verbindung der Formel IV 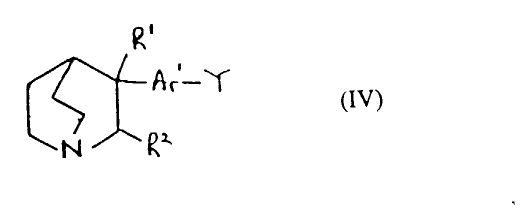 in der Y eine Abgangsgruppe ist, mit einer Verbindung der Formel IX
Ar²-CH=CH₂ (IX)
in Gegenwart eines Katalysators;
und anschließend, wenn ein pharmazeutisch verträgliches Salz verlangt wird, Umsetzen der Verbindung der Formel I mit einer Säure, die ein physiologisch verträgliches Anion zur Verfügung stellt, oder einer Base, die ein physiologisch verträgliches Kation zur Verfügung stellt ( wie es geeignet ist ).

13. Arzneimittel, das eine Verbindung der Formel I oder ein pharmazeutisch verträgliches Salz davon nach einem der Ansprüche 1 bis 11 zusammen mit einem pharmazeutisch verträglichen Verdünnungsmittel oder Träger umfaßt

14. Verwendung einer Verbindung der Formel I oder eines pharmazeutisch verträglichen Salzes davon nach einem der Ansprüche 1 bis 11 zur Herstellung eines Arzneimittels zur Behandlung von Krankheiten und medizinischen Zuständen, bei denen eine Hemmung der Cholesterinbiosynthese wünschenswert ist.

15. Verwendung nach Anspruch 14 zur Herstellung eines Arzneimittel zur Behandlung von Hypercholesterinämie oder Atherosklerose.

## Revendications

1. Composé de Formule I, ou un sel acceptable pharmaceutiquement de celui-ci, 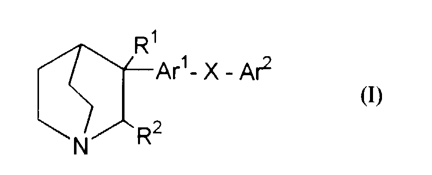 dans laquelle:
R¹ est l'hydrogène ou un groupe hydroxy;
R² est l'hydrogène;
ou R¹ et R² sont reliés ensemble de manière que CR¹-CR² soit une double liaison;
X est choisi parmi -CH₂CH₂-, -CH=CH-, -C≡C-, -CH₂O-, -OCH₂-, -CH₂NH-, -NHCH₂-, -CH₂CO-, -COCH₂-, -CH₂S-, -SCH₂-, l'atome de soufre dans les deux derniers groupes pouvant porter éventuellement un ou deux atomes d'oxygène;
Ar¹ est un segment 1,4-phénylène;
Ar² est un groupe phényle; et
dans laquelle l'un ou les deux Ar¹ et Ar² peuvent éventuellement être non substitués ou substitués indépendamment par un ou plusieurs substituants choisis parmi un groupe halogéno, hydroxy, amino, nitro, cyano, carboxy, carbamoyle, alkyle (en C1-6), alcényle (en C2-6), alcoxy (en C1-6), alkylamino (en C1-6), di-[ alkyl (en C1-6)]amino, N-[alkyl (en C1-6)] carbamoyle, N,N-di-[alkyl (en C1-6)]carbamoyle, alcoxycarbonyle (en C1-6) , alkylthio (en C1-6), alkylsulfinyle (en C1-6), alkylsulfonyle (en C1-6), halogéno-alkyle (en C1-6), et alkanoylamino (en C1-6).

2. Composé selon la revendication 1, caractérisé en ce que R¹ est l'hydrogène ou un groupe hydroxy; R² est l'hydrogène; ou R¹ et R² sont reliés ensemble de manière que CR¹-CR² soit une double liaison;
X est choisi parmi -CH₂CH₂-, -CH=CH-, -C≡C-, -CH₂O-, -OCH₂-_{,} -CH₂NH-, -NHCH₂-, -CH₂CO-, -COCH₂-, -CH₂S-, -SCH₂-, l'atome de soufre dans les deux derniers groupes pouvant porter éventuellement un ou deux atomes d'oxygène;
Ar¹ est un segment 1,4-phénylène;
Ar² est un groupe phényle; et
dans laquelle l'un ou les deux Ar¹ et Ar² peuvent éventuellement être non substitués ou substitués indépendamment par un ou plusieurs substituants choisis parmi un groupe hydroxy, amino, nitro, cyano, carboxy, carbamoyle, méthylènedioxy, isopropylènedioxy, fluoro, chloro, bromo, méthyle, éthyle, propyle, isopropyle, butyle, isobutyle, sec-butyle, allyle, but-2-ényle, 2-méthyl-2-propényle, méthoxy, éthoxy, propoxy, isopropoxy, butoxy, méthylamino, éthylamino, propylamino, butylamino, diméthylamino, diéthylamino, méthylpropylamino, dipropylamino, N-méthylcarbamoyle, N-éthylcarbamoyle, N-propylcarbamoyle, N,N-diméthyl-carbamoyle, N,N-diéthylcarbamoyle, méthoxycarbonyle, éthoxycarbonyle, propoxycarbonyle, méthylthio, éthylthio, propylthio, isopropylthio, butylthio, méthylsulfinyle, éthylsulfinyle, propylsulfinyle, isopropylsulfinyle, butylsulfinyle, méthylsulfonyle, éthylsulfonyle, propylsulfonyle, isopropylsulfonyle, butylsulfonyle, fluorométhyle, difluorométhyle, trifluorométhyle, butyryle, formamido, acétamido et propionamido.

3. Composé selon la revendication 1 ou 2, caractérisé en ce que R¹ est un groupe hydroxy et R² est l'hydrogène.

4. Composé selon l'une quelconque des revendications précédentes, caractérisé en ce que X est choisi parmi -CH₂CH₂-, -CH = CH-, -C≡C-, -CH₂O-, et -OCH₂-.

5. Composé selon la revendication 1 choisi parmi:
le 3-(4-benzyloxyphényl)quinuclidin-3-ol;
le 3-(4-phénoxyméthylphényl)quinuclidin-3-ol;
le 3-(E-4-phényléthèn-2-ylphényl)quinuclidin-3-ol;
le 3-(4-phényléthyn-2-ylphényl)quinuclidin-3-ol;
la 3-(4-benzyloxyphényl)-2,3-déshydroquinuclidine;
le 3-(4-phényléthylphényl)quinuclidin-3-ol;
et les sels acceptables pharmaceutiquement de ceux-ci.

6. Composé de Formule I, ou un sel acceptable pharmaceutiquement de celui-ci, 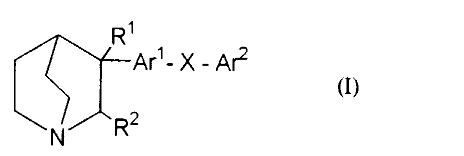 dans laquelle:
R¹ est un groupe hydroxy;
R² est l'hydrogène;
X est choisi parmi -CH₂CH₂-, -CH=CH-, -C≡C-, -CH₂O-, -OCH₂-, -CH₂NH-, -NHCH₂-, -CH₂O-, -COCH₂-, -NHCO-, -CONH-, -CH₂S-, -SCH₂-, l'atome de soufre dans les deux derniers groupes pouvant éventuellement porter un ou deux atome d'oxygène;
Ar¹ est un segment phénylène;
Ar² est un groupe phényle; et
dans laquelle l'un ou les deux Ar¹ et Ar² peuvent éventuellement être non substitués ou substitués indépendamment par un ou plusieurs substituants choisis parmi un groupe halogéno, hydroxy, amino, nitro, cyano, carboxy, carbamoyle, alkyle (en C1-6), alcényle (en C2-6), alcynyle (en C2-6), alcoxy (en C1-6), alkylamino (en C1-6), di-[ alkyl (en C1-6)]amino, N-[alkyl (en C1-6)] carbamoyle, N,N-di-[alkyl (en C1-6)]carbamoyle, alcoxycarbonyle (en C1-6) , alkylthio (en C1-6), alkylsulfinyle (en C1-6), alkylsulfonyle (en C1-6), halogénoalkyle (en C1-6), alcanoyle (en C1-6), alkylènedioxy (en C1-4) et alcanoylamino (en C1-6).

7. Composé selon la revendication 6, caractérisé en ce que R¹ est l'hydrogène ou un groupe hydroxy; R² est l'hydrogène;
X est choisi parmi -CH₂CH₂-, -CH=CH-, -C≡C-, -CH₂O-, -OCH₂-, -CH₂NH-, -NHCH₂-, -CH₂CO-, -COCH₂-, -NHCO-, -CONH-, -CH₂S-, -SCH₂-, l'atome de soufre dans les deux derniers groupes pouvant éventuellement porter un ou deux atome d'oxygène;
Ar¹ est un segment phénylène;
Ar² est un groupe phényle; et
dans laquelle l'un ou les deux Ar¹ et Ar² peuvent éventuellement être non substitués ou substitués indépendamment par un ou plusieurs substituants choisis parmi un groupe hydroxy, amino, nitro, cyano, carboxy, carbamoyle, méthylènedioxy, isopropylidènedioxy, fluoro, chloro, bromo, méthyle, éthyle, propyle, isopropyle, butyle, isobutyle, sec-butyle, allyle, but-2-ényle, 2-méthyl-2-propényle, prop-2-ynyle, but-2-ynyle, méthoxy, éthoxy, propoxy, isopropoxy, butoxy, méthylamino, éthylamino, propylamino, butylamino, diméthylamino, diéthylamino, méthylpropylamino, dipropylamino, N-méthylcarbamoyle, N-éthylcarbamoyle, N-propylcarbamoyle, N,N-diméthylcarbamoyle, N,N-diéthylcarbamoyle, méthoxycarbonyle, éthoxycarbonyle, propoxycarbonyle, méthylthio, éthylthio, propylthio, isopropylthio, butylthio, méthylsulfinyle, éthylsulfinyle, propylsulfinyle, isopropylsulfinyle, butylsulfinyle, méthylsulfonyle, éthylsulfonyle, propylsulfonyle, isopropylsulfonyle, butylsulfonyle, fluorométhyle, difluorométhyle, trifluorométhyle, formyle, acétyle, propionyle, butyryle, formamido, acétamido et propionamido.

8. Composé selon la revendication 6 ou 7, caractérisé en ce que Ar¹ est un segment 1,4-phénylène.

9. Composé selon la revendication 6, 7 ou 8, caractérisé en ce que X est choisi parmi -CH₂CH₂-, -CH=CH-, -C≡C-, -CH₂O-, -OCH₂-, et -NHCO-.

10. Composé selon la revendication 6, 7, 8 ou 9, caractérisé en ce que Ar¹ est un segment 1,4-phénylène non substitué et Ar² est un groupe phényle éventuellement substitué par un groupe alcoxy (en C1-6), hydroxy ou alkylènedioxy (en C1-4).

11. Composé selon la revendication 6, choisi parmi:
le 3-(4-[(Z)-4-méthoxystyryl]phényl)quinuclidin-3-ol;
le 3-(4-[(E)-3,4-méthylènedioxystyryl]phényl)quinuclidin-3-ol;
le 3-(4-[(E)-4-hydroxystyryl]phényl)quinuclidin-3-ol; et
le 3-(4-benzoylaminophényl)quinuclidin-3-ol;
et les sels acceptables pharmaceutiquement de ceux-ci.

12. Procédé de préparation d'un composé de Formule I, ou un sel acceptable pharmaceutiquement de celui-ci, tel que revendiqué dans la revendication 1 ou 6, caractérisé en ce que le procédé est choisi parmi:
(a) pour ceux des composés de Formule I dans laquelle les deux R¹ et R² sont l'hydrogène, la réduction d'un composé de Formule I dans laquelle R¹ et R² sont reliés ensemble de manière que CR¹-CR² soit une double liaison;
(b) pour des composés de Formule I dans laquelle R¹ et R² sont reliés ensemble de manière que CR¹ - CR² soit une double liaison, la déshydratation d'un composé de Formule I dans laquelle R¹ est un groupe hydroxy et R² est l'hydrogène;
(c) pour des composés de Formule I dans laquelle R¹ et R² sont reliés ensemble de manière que CR¹ - CR² soit une double liaison, le traitement d'un composé de Formule II 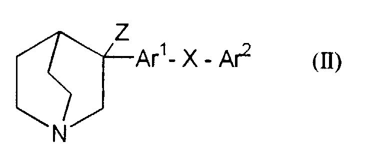 dans laquelle Z est un groupe se séparant, avec une base:
(d) pour ceux des composés de Formule I dans laquelle R¹ est un groupe hydroxy et R² est l'hydrogène, la réaction d'un dérivé organométallique de Formule III:
M-Ar¹ - X - Ar² (III)
dans laquelle M est un atome métallique ou un dérivé de celui-ci, avec une quinuclidin-3-one;
(e) pour ceux des composés de Formule I dans laquelle X est -CH = CH-, la réaction d'un composé de Formule IV: 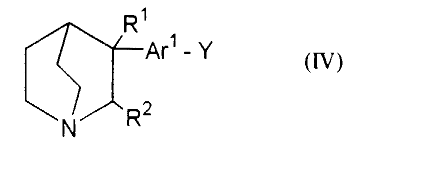 avec un composé de Formule V:
Ar²-CH₂P⁺Ph₃W⁻ (V)
dans laquelle W est un atome d'halogène, en présence d'une base;
(f) pour ceux des composés de Formule I dans laquelle X est -C ≡ C-, la réaction d'un composé de Formule IV: 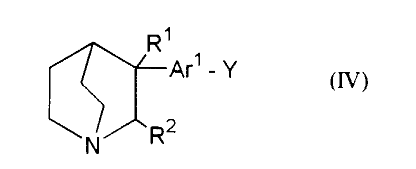 dans laquelle Y est un groupe se séparant, avec un composé de Formule VI:
Ar²- C≡CM (VI),
en présence d'un catalyseur;
(g) pour ceux des composés de Formule I dans laquelle X est -OCH₂-, -NHCH₂- ou -SCH₂-, la réaction d'un composé de Formule IV: 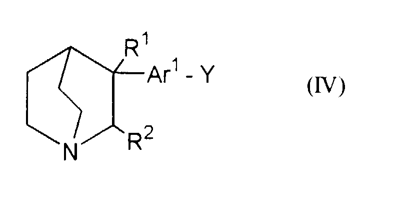 dans laquelle Y est -OH, -NH₂ ou -SH, avec un composé de Formule VII:
Ar²- CH₂Z (VII)
dans laquelle Z est un groupe se séparant, en présence d'une base;
(h) pour ceux des composés de Formule I dans laquelle X est -CH₂O-, -CH₂NH- ou -CH₂S-, la réaction avec un composé de Formule IV: 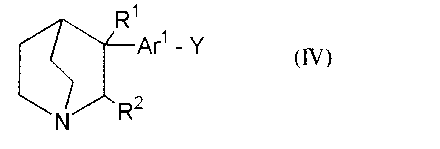 dans laquelle Y est -CH₂OH, -CH₂NH₂ ou -CH₂SH, avec un composé de Formule Ar²-Z dans laquelle Z est un groupe se séparant, en présence d'une base; et
(i) pour ceux des composés de Formule I dans laquelle X est -CH₂CH₂-, la réduction d'un composé de Formule I dans laquelle X est -C≡C- ou -CH = CH-;
(j) pour ceux des composés de Formule I dans laquelle X est -CH = CH-, la réaction d'un composé de Formule IV: 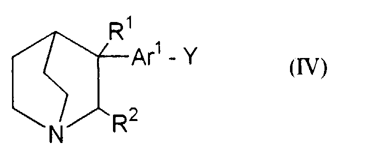 dans laquelle Y est un groupe se séparant, avec un composé de Formule IX:
Ar²-CH=CH₂ (IX),
en présence d'un catalyseur;
et ensuite, quand un sel acceptable pharmaceutiquement est requis, la réaction d'un composé de Formule I avec un acide qui donne un anion acceptable physiologiquement ou une base qui donne un cation acceptable physiologiquement (tels q'appropriés).

13. Composition pharmaceutique comprenant un composé de Formule I, ou un sel acceptable pharmaceutiquement de celui-ci, selon l'une quelconque des revendications 1 à 11, avec un diluant ou un support acceptable pharmaceutiquement.

14. Utilisation d'un composé de Formule I, ou un sel acceptable pharmaceutiquement de celui-ci, selon l'une quelconque des revendications 1 à 11, pour la fabrication d'un médicament pour le traitement de maladies et de conditions médicales selon lesquelles une inhibition de la biosynthèse du cholestérol est souhaitable.

15. Utilisation selon la revendication 14 pour la fabrication d'un médicament pour le traitement de l'hypercholestérolémie et l'artériosclérose.
